(19) Europäisches Patentamt / European Patent Office / Office européen des brevets

(11) EP 1 888 506 B1

(12) **EUROPÄISCHE PATENTSCHRIFT**

(45) Veröffentlichungstag und Bekanntmachung des Hinweises auf die Patenterteilung:
**20.03.2013 Patentblatt 2013/12**

(21) Anmeldenummer: **06753942.9**

(22) Anmeldetag: **29.05.2006**

(51) Int Cl.:
***C07C 213/10*** *(2006.01)* ***C07C 217/72*** *(2006.01)*

(86) Internationale Anmeldenummer:
**PCT/EP2006/005101**

(87) Internationale Veröffentlichungsnummer:
**WO 2006/125675 (30.11.2006 Gazette 2006/48)**

(54) **TRENNUNG STEREOISOMERER N,N-DIALKYLAMINO-2-ALKYL-3-PHENYL-ALKANE**

SEPARATION OF STEREOISOMERIC N,N-DIALKYLAMINO-2-ALKYL-3-PHENYL ALKANES

SEPARATION DE STEREOISOMERES DE N,N-DIALKYLAMINO-2-ALKYL-3-PHENYL-ALCANE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **27.05.2005 DE 102005024824**
**15.07.2005 DE 102005033732**

(43) Veröffentlichungstag der Anmeldung:
**20.02.2008 Patentblatt 2008/08**

(73) Patentinhaber: **Grünenthal GmbH**
**52078 Aachen (DE)**

(72) Erfinder:
- **BUSCHMANN, Helmut, Heinrich**
**52076 Aachen (DE)**
- **HELL, Wolfgang**
**52066 Aachen (DE)**

(74) Vertreter: **Brosch, Oliver et al**
**Kutzenberger & Wolff**
**Theodor-Heuss-Ring 23**
**50668 Köln (DE)**

(56) Entgegenhaltungen:
**EP-A- 0 693 475**

- **DEHLI J R ET AL: "Stereoselective alkylation-reduction of beta-keto nitriles by the fungus Curvularia lunata" TETRAHEDRON: ASYMMETRY, ELSEVIER SCIENCE PUBLISHERS, AMSTERDAM, NL, Bd. 12, Nr. 10, 22. Juni 2001 (2001-06-22), Seiten 1485-1492, XP004255085 ISSN: 0957-4166**
- **L. ANGIOLINI ET AL.: "STEREOCHEMISTRY OF MANNICH BASES - II. Stereospecific Synthesis and Absolute Configuration of Diastereoisomeric 1-Phenyl-1,2-Dimethyl-3-Dimethylamino-Propan-1-Ols" TETRAHEDRON., Bd. 25, 1969, Seiten 4211-4216, XP002396536 NL ELSEVIER SCIENCE PUBLISHERS, AMSTERDAM.**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 888 506 B1

Printed by Jouve, 75001 PARIS (FR)

## Beschreibung

Die Erfindung betrifft ein Verfahren zur Trennung stereoisomerer N,N-Dialkylamino-2-alkyl-3-hydroxy-3-phenylalkane.

Opioide werden seit vielen Jahren als Analgetika zur Schmerzbehandlung eingesetzt, obwohl sie eine Reihe von Nebenwirkungen, beispielsweise Sucht und Abhängigkeit, Atemdepression, gastro-intestinale Hemmwirkung und Obstipation hervorrufen können. Über einen längeren Zeitraum oder in höheren Dosierungen können sie daher nur unter besonderen Vorsichtsmaßnahmen, wie speziellen Verordnungsvorschriften, verabreicht werden.

Es ist bekannt, dass bestimmte N,N-Dialkylamino-2-alkyl-3-hydroxy-3-phenyl-alkane (insbesondere 1-Phenyt-3-dimethylamino-propan-Verbindungen) analgetisch wirksam sind, ohne dabei die für Opioide typischen Nebenwirkungen hervorzurufen. Diese Substanzen zeichnen sich durch eine ausgeprägte analgetische Wirkung aus, die beispielsweise im Vergleich zum Opioid Tramadol deutlich verstärkt ist.

Diese analgetisch wirksamen N,N-Dialkylamino-2-alkyl-3-hydroxy-3-phenyl-alkane sind chiral. Da meist zwei Chiralitätszentren vorhanden sind, kommen die Verbindungen in Form von 4 Stereoisomeren vor, d.h. zwei Enantiomerenpaaren, die untereinander diastereomer sind.

Die Wirksamkeit eines Analgetikums ist häufig auf seine Wechselwirkung mit einem bestimmten Rezeptor einer menschlichen oder tierlschen Zelle zurückzuführen. Da diese Rezeptoren aus chiralen Aminosäuren und ggf. auch chiralen Glycosiden aufgebaut sind, erfolgt ihre Wechselwirkung mit chiralen Arzneistoffen stereoselektiv. Daher ist die pharmakologische Wirksamkeit chiraler Arzneistoffe häufig für die einzelnen Stereoisomere unterschiedlich.

Die stereoselektive Gewinnung von $\alpha$-Alkyl-$\beta$-hydroxynitrilen aus $\beta$-Ketonitrilen durch biokatalytische Einführung einer Alkylgruppe und Reduktion der Carbonylgruppe wurde von J. R. Dehli et al. beschrieben (Tetrahedron: Asymmetry, Vol. 12, Nr. 10, 2001, Seiten 1485-1492). Die chiralen $\alpha$-Alkyl-$\beta$-hydroxynitrile können als Vorläufer für die Gewinnung von Aminoalkoholen eingesetzt werden.

Die stereoscezifische Synthese von (±)-1-Phenyl-1,2-dimethyl-3-dimethylaminoprooan-1-olen durch Reaktion eines geeigneten Grignard-Reagenzes mit $\alpha$-Methyl-$\beta$-dimethylamino-propiophenon oder 3-Methyl-4-dimethylaminobutan-2-on wurde von L. Angliolini et al. beschreiben (Tetrahedron, Vol. 25, 1969, Seiten 4211-4216).

Keines dieser beiden Verfahren beinhaltet die Auftrennung von Stereoisomerenmischungen.

Es besteht daher ein Bedarf an einem Verfahren zur Trennung der Stereoisomere, d.h. der Diastereomere und Enantiomere, von N,N-Dialkylamino-2-alkyl-3-hydroxy-3-phenyl-alkanen.

Aus EP-A 693 475 ist die Synthese von (2RS,3RS)-1-Dimethylamino-3-(3-methoxyphenyl)-2-methyl-pentan-3-ol bekannt Die Trennung der Diastereomere, d.h. der beiden Enantiomerenpaare, erfolgt durch Hydrochloridfällung mit Trimethylchlorsilan/Wasser in 2-Butanon. Die racemische Mischung der beiden Enantiomere der Konfiguration (2R,3R) und (2S,3S) erfolgt durch Trennung an einer chiralen HPLC-Säule.

Die chromatographische Trennung von Enantiomeren an chiralen stationären Phasen ist jedoch in der Regel nicht für größere Wirkstoffmengen geeignet, sondern dient meist ausschließlich analytischen Zwecken. Es besteht daher ein Bedarf an einem Verfahren, welches auch zur Trennung der Enantiomere chiraler N,N-Dialkylamino-2-alkyl-3-hydroxy-3-phenyl-alkane in präparativem Maßstab geeignet ist.

Der Erfindung liegt somit die Aufgabe zugrunde, ein Verfahren zur Trennung der Stereoisomere, vorzugsweise Enantiomere, chiraler N,N-Dialkylamino-2-alkyl-3-hydroxy-3-phenyl-alkane bereitzustellen, welches auch im Gramm- und Kilogrammmaßstab durchgeführt werden kann. Das Verfahren sollte kostengünstig sein, eine gute Ausbeute und hohe Enantiomerenreinheit gewährleisten.

Diese Aufgabe wird durch den Gegenstand der Patentansprüche gelöst. Es wurde überraschend gefunden, dass die Isolierung eines Stereoisomers aus einer Mischung umfassend die beiden Stereoisomere der allgemeinen Formeln (I-A) und (I-A') und/oder die beiden Stereoisomere der allgemeinen Formeln (I-B) und (I-B'),

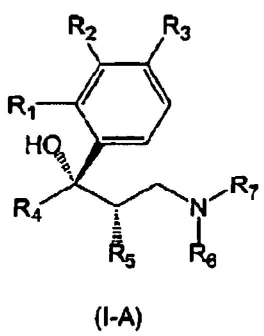
(I-A)

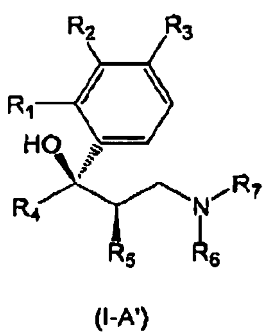
(I-A')

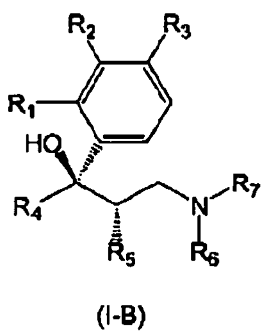
(I-B)

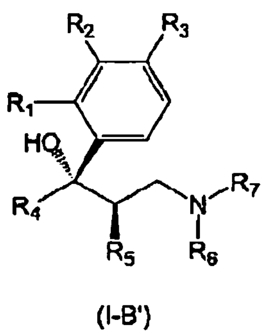
(I-B')

worin

$R_1$, $R_2$ und $R_3$ gleich oder verschieden ausgewählt sind aus der Gruppe bestehend aus -H, -F, -Cl, -$C_1$-$C_6$-alkyl, -S-$C_1$-$C_6$-alkyl, -OH, -O-$C_1$-$C_6$-alkyl, -O-$C_1$-$C_6$-alkylen-phenyl, -OCO-$C_1$-$C_6$-alkyl, -OCON($C_1$-$C_6$-alkyl)$_2$ und -O-$SiR_8R_9R_{10}$ (worin $R_8$, $R_9$ und $R_{10}$ gleich oder verschieden -$C_1$-$C_6$-alkyl oder -Phenyl sind);
$R_4$ -H oder -$C_1$-$C_6$-alkyl ist;
$R_5$ -$C_1$-$C_6$-alkyl ist; und
$R_6$ und $R_7$ gleich oder verschieden -H oder -$C_1$-$C_6$-alkyl sind;
wobei
"$C_1$-$C_6$-alkyl" einen linearen oder verzweigten, cyclischen oder offenkettigen, gesättigten Kohlenwasserstoffrest mit 1 bis 6 Kohlenstoffatomen, ggf. mit 1 bis 6 Halogenatomen, gleich oder verschieden ausgewählt aus -F, -Cl und -Br, substituiert, bedeutet, und "$C_1$-C6-alkylen" einen linearen oder verzweigten, cyclischen oder offenkettige, gesättigten Kohlenwasserstoffrest mit 1 bis 6 Kohlenstoffatomen, ggf. mit 1 bis 6 Halogenatomen. gleich oder verschieden ausgewählt aus -F, -Cl und -Br, substituiert, bedeutet:

oder deren Salze mit organischen oder anorganischen Säuren;
möglich ist durch ein Verfahren umfassend die Schritte

(a) Beeinflussen des MIschungsverhältnisses der Stereoisomere in der Mischung, so dass zumindest eines der Stereoisomere, vorzugsweise das zu isolierende Stereoisomer, mit einem Enantiomerenüberschuss vorliegt, und

(b) Zugeben von Phosphorsäure unter Bedingungen, welche die Präzipitation des im Enantiomerenüberschuss vorliegenden Stereoisomers aus einer Lösung als Phosphat, Hydrogenphosphat oder Dihydrogenphoshat bewirken.

Es wurde überraschend gefunden, dass sich einzelne Stereoisomere mit hoher Stereoselektivität aus der Mischung der Stereoisomere auskristallisieren lassen, wenn zumindest eines der Stereoisomere nicht im racemischen Gemisch, sondern mit einem Enantiomerenüberschuss vorliegt.

Die Verbindungen der allgemeinen Formel (I) weisen mindestens zwei Chiralitätszentren auf. Die Verbindungen können auf unterschiedliche Weise synthetisiert werden. Beispielsweise ist es möglich, zunächst in einer Mannich-Reaktion eine Mannich-Base herzustellen, wodurch das erste Chiralitätszentrum erzeugt wird:

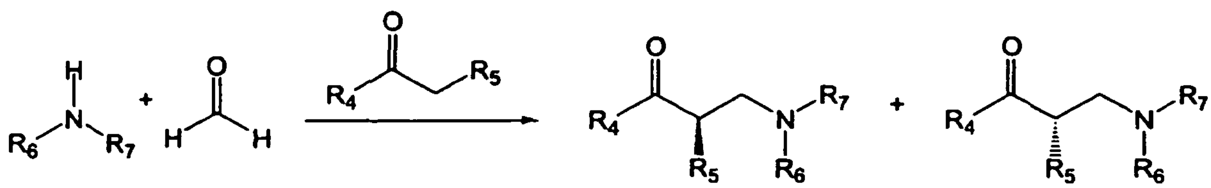

Erfolgt die Mannich-Reaktion ohne chirale Induktion, d.h. in Abwesenheit chiraler Auxiliare, chiraler Katalysatoren, chiraler Lösungsmittel, etc., so entsteht die Mannich-Base als racemisches Gemisch, da die zu den beiden enantiomeren Mannich-Basen führenden Übergangszustände enantiomorph und somit energetisch gleich sind.

Das zweite Chiralitätszentrum kann beispielsweise eingeführt werden, indem die Mannich-Base am Carbonyl mit einem Grignard-Reagens umgesetzt wird:

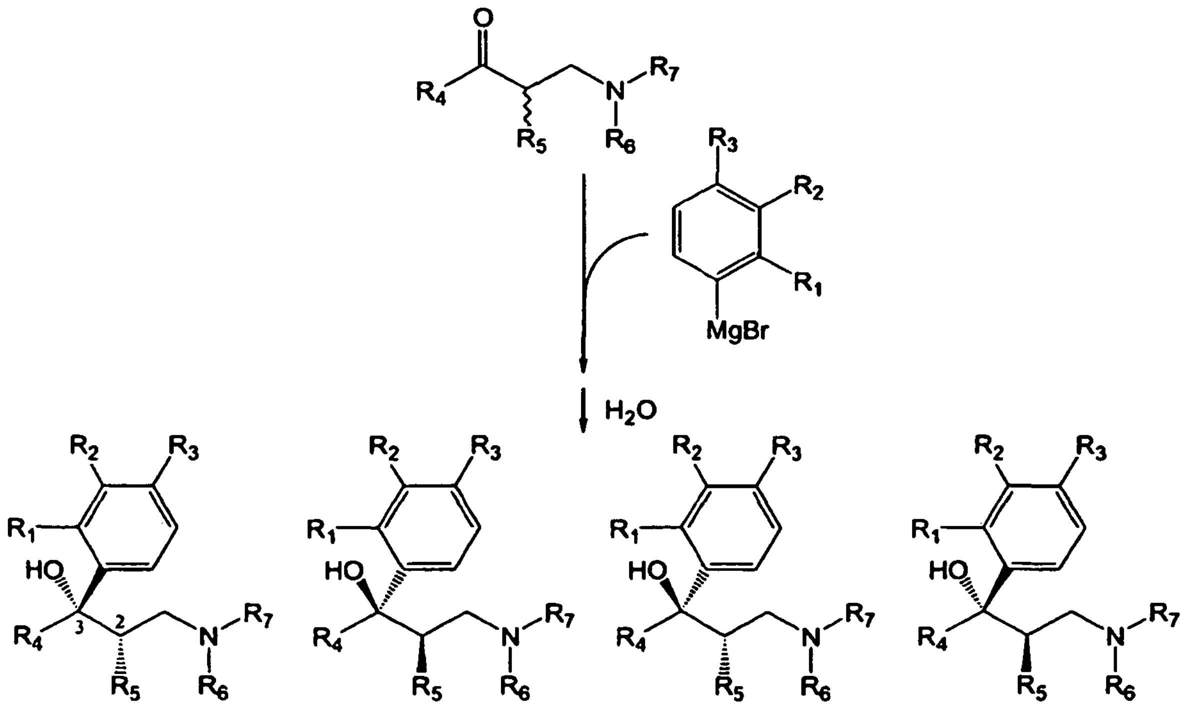


(I-A) (I-A') (I-B) (I-B')

Auf diese Weise entstehen aus der racemischen Mannich-Base die vier stereoisomeren N,N-Dialkylamino-2-alkyl-3-hydroxy-3-phenyl-alkane der allgemeinen Formeln (I-A), (I-A'), (I-B) und (I-B'). Je nach Substitutionsmuster an den zwei Chiralitätszentren ergibt sich jeweils die R- oder S-Konfiguration.

Grundsätzlich ist es auch möglich, dass weitere Chiralitätszentren in einer der Seitenketten vorhanden sind. Im Fall dreier Chiralitätszentren erhöht sich die Zahl der Stereoisomere auf insgesamt acht, wobei vier Enantiomerenpaare vorhanden sind. Bevorzugt weisen die N,N-Dialkylamino-2-alkyl-3-hydroxy-3-phenyl-alkane nur zwei Chiralitätszentren auf.

Bevorzugt sind die Stereoisomere (I-A) und (I-A') einerseits und die Stereoisomere (I-B) und (I-B') andererseits jeweils zueinander enantiomer. Untereinander sind die Stereoisomere ansonsten diastereomer. Vorzugsweise ist demnach Stereoisomer (I-A) zu (I-A') enantiomer und zu (I-B) und (I-B') diastereomer, Stereoisomer (I-A') zu (I-A) enantiomer und zu (I-B) und (I-B') diastereomer, Stereoisomer (I-B) zu (I-A) und (I-A') diastereomer und zu (I-B') enantiomer, und schließlich Stereoisomer (I-B') zu (I-A) und (I-A') diastereomer und zu (I-B) enantiomer.

Wird in Schritt (a) des erfindungsgemäßen Verfahrens beispielsweise das Mischungsverhältnis der Stereoisomere in der Mischung derart beeinflusst, so dass das Stereoisomer der allgemeinen Formel (I-A') mit einem Enantiomerenüberschuss vorliegt, so bezieht sich der Überschuss relativ auf die Menge seines Enantiomers, d.h. vorzugsweise auf das Stereoisomer der allgemeinen Formel (I-A). Das Mischungsverhältnis der ggf. ebenfalls anwesenden Stereoisomere, wie beispielsweise der allgemeinen Formeln (I-B) und (I-B'), braucht nicht notwendigerweise ebenfalls beeinflusst zu werden, so dass diese z.B. auch (ggf. weiterhin) im racemischen Gemisch vorliegen können.

In einer bevorzugten Ausführungsform des erfindungsgemäßen Verfahrens dient dieses zur Isolierung eines Stereoisomers aus einer Mischung umfassend die beiden Stereoisomere der allgemeinen Formeln (I-A) und (I-A'), nicht jedoch die beiden Stereoisomere der allgemeinen Formeln (I-B) und (I-B'). In einer anderen bevorzugten Ausführungsform des erfindungsgemäßen Verfahrens dient dieses zur Isolierung eines Stereoisomers, vorzugsweise der allgemeinen Formel (I-A) oder (I-A'), aus einer Mischung aller vier Stereoisomere, d.h. der Stereoisomere der allgemeinen Formeln (I-A), (I-A'), (I-B) und (I-B').

In einer bevorzugten Ausführungsform des erfindungsgemäßen Verfahrens erfolgt die Beeinflussung in Schritt (a)

- durch Zugabe des Stereoisomers, welches im Überschuss vorliegen soll, oder
- durch enantioselektive Synthese der Stereoisomere.

Die Beeinflussung in Schritt (a) hat zur Folge, dass wenigstens eines der Stereoisomere mit einem Enantiomerenüberschuss vorliegt. Dies bedeutet vorzugsweise, dass es relativ im Vergleich zu seinem Enantiomer angereichert

wird. Diese Anreicherung kann auf verschiedene Weise erreicht werden. Einerseits umfasst sie die Erzielung eines Enantiomerenüberschusses bereits im Wege der Synthese der Stereoisomere, andererseits umfasst sie aber auch die Verschiebung des relativen Gewichtsverhältnisses der beiden Enantiomere eines ursprünglich hinsichtlich dieser beiden Stereoisomere/Enantiomere als Racemat vorliegenden Gemisches. Letztere Variante kann z.B. durch Zugabe von außen oder durch enantioselektive Derivatisierung eines der beiden Enantiomere (z.B. kinetische Racematspaltung) verwirklicht werden.

Erfolgt die Beeinflussung in Schritt (a) durch Zugabe des Stereoisomers, welches im Enantiomerenüberschuss vorliegen soll, so kann dieses zuvor durch separate Isolierung aus einer Mischung der Stereoisomere gewonnen werden. Beispielsweise ist es dazu möglich, zunächst die Diastereomere durch selektive Fällung des einen Enantiomerenpaars als Hydrochloride abzutrennen. Auf diese Weise lassen sich die beiden Stereoisomere der allgemeinen Formel (I-A) und (I-A') (Enantiomerenpaar 1) von den beiden Stereoisomeren der allgemeinen Formel (I-B) und (I-B') (Enantiomerenpaar 2) abtrennen. Die Trennung der beiden Enantiomere, d.h. die Spaltung der racemischen Enantiomerenpaare 1 bzw. 2, kann dann mit Hilfe chiraler Hilfsreagenzien, beispielsweise chiraler Säuren, erfolgen.

Es wurde überraschend gefunden, dass sich die beiden Enantiomere der allgemeinen Formel (I-A) und (I-A') selektiv mit Hilfe von (+)- bzw. (-)-Di-O,O'-p-toluyl-weinsäure präzipitieren lassen, wobei das jeweils andere Enantiomer in der Mutterlauge zurückbleibt.

Ein Aspekt der Erfindung betrifft somit ein Verfahren zur Racematspaltung der beiden Enantiomere der allgemeinen Formeln (I-A) und (I-A') umfassend die Zugabe von (+)- oder (-)-Di-O,O'-p-toluyl-weinsäure. Als Lösungsmittel eignet sich beispielsweise 2-Butanon. Die beiden Enantiomere der allgemeinen Formeln (I-A) und (I-A') werden dabei in dem Lösungsmittel gelöst. Je nach Konfiguration des verwendeten Weinsäurederivats erfolgt nach dessen Zugabe eine stereoselektive Präzipitation des Enantiomers der allgemeinen Formel (I-A) bzw. (I-A') als Additionssalz.

Aufgrund der vergleichsweise hohen Kosten des enantiomerenreinen Weinsäurederivats ((-)- bzw. (+)-Di-O, O'-p-toluyl-weinsäure) als chirales Hilfsreagenz ist dieses Verfahren jedoch nur bedingt für die Enantiomerentrennung in präparativem Maßstab geeignet. Bevorzugt wird dieses Verfahren lediglich dazu verwendet, zunächst jeweils die enantiomerenreinen Stereoisomere der allgemeinen Formel (I-A) und (I-A') zu erhalten und durch deren Zusatz zur Reaktionsmischung das relative Gewichtsverhältnis im Sinne von Schritt (a) des erfindungsgemäßen Verfahrens zugunsten des einen oder des anderen Stereoisomers zu beeinflussen, d.h. den Enantiomerenüberschuss zu erzeugen.

Alternativ kann dazu eine geringe, aber ausreichende Menge der enantiomerenreinen Verbindungen durch HPLC an einer chiralen stationären Phase erhalten werden.

Grundsätzlich ist es auch denkbar, dass die Beeinflussung in Schritt (a) des erfindungsgemäßen Verfahrens nicht durch Zugabe des einen Enantiomers, sondern durch selektive Entfernung des anderen Enantiomers erfolgt. So kann beispielsweise alternativ zur Anreicherung eines der beiden Enantiomere auch ein selektiver Abbau eines der beiden Enantiomere erfolgen. Denkbar ist in diesem Zusammenhang beispielsweise die selektive, enzymatische Derivatisierung eines Stereoisomers, wodurch sein Enantiomer im Überschuss vorliegt.

Die Beeinflussung in Schritt (a) des erfindungsgemäßen Verfahrens kann auch durch enantioselektive Synthese der Stereoisomere erfolgen. Dabei kann die Enantioselektivität auf verschiedenen Synthesestufen induziert werden. So ist es beispielsweise möglich, bereits auf der Stufe der bereits erwähnten Mannich-Reaktion durch geeignete Maßnahmen einen Überschuss des einen der beiden entstehenden Enantiomere zu induzieren. Geeignete Maßnahmen sind dem Fachmann bekannt. Alternativ ist es beispielsweise möglich, durch enantioselektive Reaktionsführung der sich an die Mannich-Reaktion anschließenden, ebenfalls bereits erwähnten Grignard-Reaktion (oder gleichwirkenden Umsetzung mit einem anderen metallorganischen Reagens), einen Enantiomerenüberschuss zu induzieren. Geeignete Maßnahmen sind auch hier dem Fachmann bekannt. In diesem Zusammenhang kann beispielsweise vollumfänglich verwiesen werden auf I. Ojima, Catalytic Asymmetric Synthesis, 2nd ed., Wiley VCH; R.A. Aitken et al., Asymmetric Synthesis, 2nd ed., CRC Press; L.A. Paquette, Handbook of Reagents for Organic Synthesis : Chiral Reagents for Asymmetric Synthesis, John Wiley & Sons; H.B. Kagan, Asymmetric Synthesis, Thieme Medical Pub.; W. Carruthers, Modem Methods of Organic Synthesis, 4th ed., Cambridge University Press; und R.S. Atkinson, Stereoselective Synthesis, John Wiley & Sons.

In einer bevorzugten Ausführungsform beträgt der Enantiomerenüberschuss nach der Durchführung von Schritt (a) des erfindungsgemäßen Verfahrens wenigstens 1,0%ee, bevorzugter wenigstens 2,5%ee, noch bevorzugter wenigstens 5,0%ee, am bevorzugtesten wenigstens 7,5%ee und insbesondere wenigstens 10%ee. Bevorzugt beträgt der Enantiomerenüberschuss jedoch höchstens 25%ee, bevorzugter höchstens 20%ee und insbesondere höchstens 15%ee.

Der Enantiomerenüberschuss ist definiert als $\frac{[(+)]-[(-)]}{[(+)]+[(-)]}$ 100 % bzw. $\frac{[(-)]-[(+)]}{[(+)]+[(-)]}$ 100%, wobei [(+)] und [(-)] die Konzentration des rechts- und des linksdrehenden Enantiomers bedeuten und stets ein positiver Wert erhalten wird.

Geeignete Methoden zur Bestimmung des Enantiomerenüberschusses sind dem Fachmann geläufig. Als Beispiele können HPLC an chiralen stationären Basen und NMR-Untersuchungen mit chiralen *Shift*-Reagenzien genannt werden.

Das erfindungsgemäße Verfahren gelingt mit N,N-Dialkylamino-2-alkyl-3-hydroxy-3-phenyl-alkanen unterschiedlicher Struktur.

In einer bevorzugten Ausführungsform sind $R_1$ und $R_3$ -H. Bevorzugte Substitutionsmuster des Phenylrings ($R_1$, $R_2$ und $R_3$) sind in nachfolgender Tabelle zusammengefasst:

| $R_1$ | $R_2$ | $R_3$ | | $R_1$ | $R_2$ | $R_3$ |
|---|---|---|---|---|---|---|
| -H | $-OCH_3$ | -H | | -H | $-CF_3$ | -H |
| -H | -OH | -H | | -H | -H | $-CF_3$ |
| -H | $-OCH(CH_3)_2$ | -H | | -H | -Cl | -H |
| -H | $-OCH_2C_6H_5$ | -H | | -H | -F | -H |
| -H | $-CH_3$ | -H | | -H | -Cl | -Cl |
| -H | $-CHF_2$ | -H | | -H | $-SCH_3$ | -H |

Besonders bevorzugt sind $R_1$ und $R_3$ jeweils -H und $R_2$ ist $-O-C_1-C_6$-alkyl, vorzugsweise $-OCH_3$, oder -OH. $R_4$ ist bevorzugt $-C_1-C_6$-alkyl, vorzugsweise $-CH_3$ oder $-CH_2CH_3$. $R_5$ ist bevorzugt $-C_1-C_6$-alkyl, vorzugsweise $-CH_3$. $R_6$ und $R_7$ sind bevorzugt jeweils $-C_1-C_6$-alkyl, vorzugsweise $-CH_3$. Besonders bevorzugt ist $R_4$ $-CH_2CH_3$ und $R_5$, $R_6$ und $R_7$ sind jeweils $-CH_3$.

"$C_1-C_6$-alkyl" im Sinne der Beschreibung bedeutet einen linearen oder verzweigten, cyclischen oder offenkettigen, gesättigten Kohlenwasserstoffrest mit 1 bis 6 Kohlenstoffatomen, ggf. mit 1 bis 6 Halogenatomen (gleich oder verschieden ausgewählt aus -F, -Cl und -Br) substituiert. Beispiele sind $-CH_3$, $-CF_3$, $-CH_2F$, $-CHF_2$, $-CH_2CH_3$, $-CH(CH_3)_2$. $-CH_2CH_2CH_3$, $-CH(CH_3)CH_2CH_3$, $-CH_2CH(CH_3)CH_3$, $-C(CH_3)_3$, $-CH_2CH_2CH_2CH_3$, etc.

"$C_1-C_6$-alkylen" im Sinne der Beschreibung bedeutet einen linearen oder verzweigten, cyclischen oder offenkettigen, gesättigten Kohlenwasserstoffrest mit 1 bis 6 Kohlenstoffatomen, ggf. mit 1 bis 6 Halogenatomen (gleich oder verschieden ausgewählt aus -F, -Cl und -Br) substituiert. Beispiele sind $-CH_2-$, $-CH_2CH_2-$,- $CH(CH_3)CH_2-$, $-CH_2CH_2CH_2-$, $-CH(CH_3)CH_2CH_2-$, $-CH_2CH(CH_3)CH_2-$, $-C(CH_3)_2CH_2-$, $-CH(CH_3)CH(CH_3)-$, $-CH_2CH_2CH_2CH_2-$, etc.

In einer besonders bevorzugten Ausführungsform des erfindungsgemäßen Verfahrens ist $R_1$ -H, $R_2$-$OCH_3$, $R_3$-H, $R_4$-$CH_2CH_3$, $R_5$-$CH_3$, $R_8$-$CH_3$ und $R_7$-$CH_3$, so dass es sich bei den Stereoisomeren der allgemeinen Formeln (I-A), (I-A'), (I-B) und (I-B') um 1-Dimethylamino-3-(3-methoxy-phenyl)-2-methyl-pentan-3-ol handelt.

In diesem Fall handelt es sich bei dem Stereoisomer der allgemeinen Formel (I-A) um (2S,3S)-1-Dimethylamino-3-(3-methoxy-phenyl)-2-methyl-pentan-3-ol, bei dem Stereoisomer der allgemeinen Formel (I-A') um (2R,3R)-1-Dimethylamino-3-(3-mothoxy-phonyl)-2-methyl-pentan-3-ol, bei dem Stereoisomer der allgemeinen Formel (I-B) um (2S,3R)-1-Dimethylamino-3-(3-methoxy-phenyl)-2-methyl-pentan-3-ol, und bei dem Stereoisomer der allgemeinen Formel (I-B') um (2R,3S)-1-Dimethylamino-3-(3-methoxy-phenyl)-2-methyl-pentan-3-ol, bzw. um deren Salze mit organischen oder anorganischen Säuren.

Die Zugabe von Phosphorsäure in Schritt (b) des erfindungsgemäßen Verfahrens dient bevorzugt dazu, zumindest eines der Stereoisomere in das Phosphat, Hydrogenphosphat bzw. Dihydrogenphosphat zu überführen und dadurch selektiv zur Auskristallisation zu bringen. Ober das stöchiometrische Verhältnis zwischen der zugesetzten Menge an Phosphorsäure und den Stereoisomeren der allgemeinen Formeln (I-A), (I-A'), (I-B) und (I-B') kann Einfluss darauf genommen werden, ob die Salzbildung als Phosphat ($PO_4^{3-}$), als Hydrogenphosphat ($HPO_4^{2-}$) oder als Dihydrogenphosphat ($H_2PO_4^-$) erfolgt.

Neben der Zugabe einer geeigneten Menge von Phosphorsäure können die Stereoisomere auch auf andere Weise in die Phosphate, Hydrogenphosphate bzw. Dihydrogenphosphate überführt werden. Derartige Varianten sind dem Fachmann bekannt, beispielsweise die Zugabe von Phosphatsalzen und anschließendes Ansäuern mit Mineralsäure.

Mit der Zugabe von Phosphorsäure in Schritt (b) des erfindungsgemäßen Verfahrens wird kein weiteres chirales Reagenz hinzugegeben. Stattdessen basiert die Trennung der Stereoisomere vorzugsweise auf dem unterschiedlichen Kristallisationsverhalten der Phosphate, Hydrogenphosphate bzw. Dihydrogenphosphate der Stereoisomere als solche. Es wurde überraschend gefunden, dass aus einer Mischung der beiden Enantiomere der allgemeinen Formel (I-A) und (I-A') das eine Enantiomer selektiv als Phosphat, Hydrogenphosphat bzw. Dihydrogenphosphat präzipitiert werden kann, wenn zuvor dafür gesorgt wurde, dass dieses in einem gewissen, jedoch nicht notwendigerweise besonders großen Enantiomerenüberschuss vorliegt.

Das erfindungsgemäße Verfahren eignet sich sogar für die selektive Auskristallisation eines der Stereoisomere aus dem Gemisch aller vier Stereoisomere der allgemeinen Formeln (I-A), (I-A'), (I-B) und (I-B'). Daher ist es nicht einmal erforderlich, zunächst eine Diastereomerentrennung, beispielsweise durch Präzipitation des einen Enantiomerenpaars als Hydrochlorid, durchzuführen. Stattdessen gelingt die selektive Fällung beispielsweise sogar aus dem unmittelbaren Reaktionsprodukt der Grignard-Reaktion. Als Lösungsmittel eignet sich dafür beispielsweise Ethanol.

Das erfindungsgemäße Verfahren hat den Vorteil, dass in Schritt (a) nur ein vergleichsweise geringer Enantiomerenüberschuss des Stereoisomers bewirkt werden muss, um bei der Präzipitation als Phosphat Hydrogenphosphat bzw. Dihydrogenphosphat einen hohen Enantiomerenüberschuss des Präzipitats zu erreichen. Wird beispielsweise Schritt (a) des erfindungsgemäßen Verfahrens durch enantioselektive Reaktionsführung durchgeführt, muss die Enantioselektivität der Reaktion daher nicht wie sonst üblicherweise gefordert im Bereich von 95%ee und darüber liegen. Stattdessen reichen bereits deutlich geringer ausgeprägte Enantioselektivitäten bei der Synthese aus, um letztendlich ein Produkt mit sehr hoher Enantiomerenreinheit zu erhalten.

Bevorzugt umfasst das erfindungsgemäße Verfahren den Schritt

(c) Abtrennen des in Schritt (b) erhaltenen präzipitierten Salzes von der überstehenden Lösung.

Dies kann beispielsweise durch Dekantieren, Filtrieren, Zentrifugieren, etc. erreicht werden.

In einer bevorzugten Ausführungsform umfasst das erfindungsgemäße Verfahren den Schritt

(d) Überführen des in Schritt (c) abgetrennten Salzes in das Hydrochlorid.

Die Überführung des abgetrennten Salzes in das Hydrochlorid kann auf unterschiedliche Weise realisiert werden. In einer bevorzugten Ausführungsform wird das abgetrennte Salz zunächst In die frele Base des Stereoisomers überführt. Dies kann durch Zusatz starker Basen, beispielsweise von NaOH oder KOH, geschehen.

Die auf diese Weise freigesetzte freie Base des Stereoisomers kann nun entweder zunächst isoliert oder aber direkt *in situ* in das Hydrochlorid überführt werden. Es wurde überraschend gefunden, dass sich verbesserte Ausbeuten bei der Hydrochloridfällung erreichen lassen, wenn anstelle von Trimethylchlorsilan-Wasser (vgl. EP-A 693 475) Chlorwasserstoff als Gas eingesetzt wird. Als Lösungsmittel eignen sich dafür beispielsweise Aceton oder 2-Butanon.

Ein weiterer Aspekt der Erfindung betrifft ein Additionssalz eines Stereoisomers der allgemeinen Formel (I-A), (I-A'), (I-B) oder (I-B'), wie vorstehend definiert, und

- Phosphorsäure.

Bevorzugt ist das Additionssalz ausgewählt aus der Gruppe bestehend aus

- (2R,3R)-1-Dimethylamino-3-(3-methoxy-phenyl)-2-methyl-pentan-3-ol-phosphat,
- (2R,3R)-1-Dimethylamino-3-(3-methoxy-phenyl)-2-methyl-pentan-3-ol-hydrogenphosphat,
- (2R,3R)-1-Dimethylamino-3-(3-methoxy-phenyl)-2-methyl-penten-3-ol-dihydrogenphosphat,
- (2S,3S)-1-Dimethylamino-3-(3-methoxy-phenyl)-2-methyl-pentan-3-ol-phosphat,
- (2S,3S)-1-Dimethylamino-3-(3-methoxy-phenyl)-2-methyl-pentan-3-ol-hydrogenphosphat,
- (2S,3S)-1-Dimethylamino-3-(3-methoxy-phenyl)-2-methyl-pentan-3-ol-dihydrogenphosphat,
- (2R,3S)-1-Dimethylamino-3-(3-methoxy-phenyl)-2-methyl-pentan-3-ol-phosphat,
- (2R,3S)-1-Dimethylamino-3-(3-methoxy-phenyl)-2-methyl-pentan-3-ol-hydrogenphosphat,
- (2R,3S)-1-Dimethylamino-3-(3-methoxy-phenyl)-2-methyl-pentan-3-ol-dihydrogenphosphat,
- (2S,3R)-1-Dimethylamino-3-(3-methoxy-phenyl)-2-methyl-pentan-3-ol-phosphat,
- (2S,3R)-1-Dimethylamino-3-(3-methoxy-phenyl)-2-methyl-pentan-3-ol-hydrogenphosphat und
- (2S,3R)-1-Dimethylamino-3-(3-methoxy-phenyl)-2-methyl-pentan-3-ol-dihydrogenphosphat.

In einer bevorzugten Ausführungsform ist das Additionssalz ausgewählt aus der vorstehenden Liste, wobei jeweils "(3-methoxy-phenyl)" durch "(3-hydroxy-phenyl)" ersetzt ist.

Ein weiterer Aspekt der Erfindung betrifft ein Verfahren zur Herstellung eines Stereoisomers der allgemeinen Formel (I-A), (I-A'), (I-B) und (I-B') wie vorstehend definiert (Herstellungsverfahren), umfassend das vorstehend beschriebene Verfahren zur Isolierung des Stereoisomers (Trennverfahren). Bevorzugt handelt es sich bei dem Herstellungsverfahren um ein Verfahren zur Herstellung eines Stereoisomers der allgemeinen Formel (I-A), (I-A'), (I-B) oder (I-B')

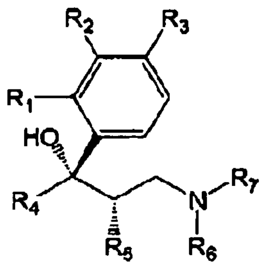

(I-A)

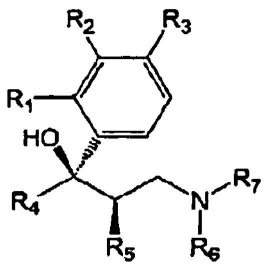

(I-A')

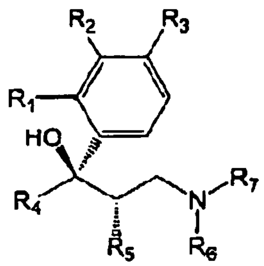

(I-B)

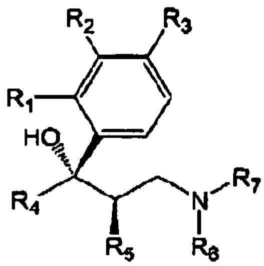

(I-B')

worin
$R_1$, $R_2$ und $R_3$ gleich oder verschieden ausgewählt sind aus der Gruppe bestehend aus -H, -F, -Cl, -$C_1$-$C_6$-alkyl, -S-$C_1$-$C_6$-alkyl und -OH, mit der Maßgabe, dass zumindest einer der Reste $R_1$, $R_2$ und $R_3$ -OH ist; $R_4$ -H oder -$C_1$-$C_6$-alkyl ist; $R_5$ -$C_1$-$C_6$-alkyl ist; und $R_6$ und $R_7$ gleich oder verschleden -H oder -$C_1$-$C_6$-alkyl sind; oder deren Salze mit organischen oder anorganischen Säuren; umfassend das vorstehend beschriebene Trennverfahren zur Isolierung eines Stereoisomers, wobei während der Durchführung des Trennverfahrens zumindest einer der Reste $R_1$, $R_2$ und $R_3$ -O-$C_1$-$C_6$-alkyl, -O-$C_1$-$C_6$-alkylen-phenyl, -OCO-$C_1$-$C_6$-alkyl, -$OCO_2$-$C_1$-$C_6$-alkyl, -OCON($C_1$-$C_6$-alkyl)$_2$ oder -O-$SiR_8R_9R_{10}$ ist;
wobei
"$C_1$-$C_6$-alkyl" einen linearen oder verzweigten, cyclischen oder offenkettigen, gesättigten Kohlenwasserstoffrest mit 1 bis 6 Kohlenstoffatomen, ggf. mit 1 bis 6 Halogenatomen, gleich oder verschieden ausgewählt aus -F, -Cl und -Br, substituiert, bedeutet, und
"$C_1$-$C_6$-alkylen" einen linearen oder verzweigten, cyclischen oder offenkettigen, gesättigten Kohlenwasserstoffrest mit 1 bis 6 Kohlenstoffatomen, ggf. mit 1 bis 6 Halogenatomen, gleich oder verschieden ausgewählt aus -F. - Cl und -Br, substituiert, bedeutet.

In einer bevorzugten Ausführungsform umfasst das erfindungsgemäße Herstellungsverfahren nach der Isolierung des Stereoisomers, d.h. nach der Durchführung des erfindungsgemäßen Trennverfahrens, den Schritt

(e) Überführen des zumindest einen der Reste $R_1$, $R_2$ und $R_3$, welcher -O-$C_1$-$C_6$-alkyl, -O-$C_1$-$C_6$-alkylen-phenyl, -OCO-$C_1$-$C_6$-alkyl, -$OCO_2$-$C_1$-$C_6$-alkyl, -OCON($C_1$-$C_6$-alkyl)$_2$ oder -O-$SiR_8R_9R_{10}$ ist, in eine -OH Gruppe.

Die Überführung in eine -OH Gruppe kann auf dem Fachmann bekannte Weise erfolgen, je nach Substitution beispielsweise mit Säure, Base, Fluorid, komplexen Metallhydriden, etc.

Die nachfolgenden Beispiele dienen zur Illustration der Erfindung, sind jedoch nicht einschränkend hinsichtlich ihres Umfangs auszulegen.

__Beispiel 1__

Herstellung einer Mischung der vier Stereoisomere von 1-Dimethylamino-3-(3-methoxyphenyl)-2-methylpentan-3-ol:

Gemäß Beispiel 1 von EP 0 693 475 A1 wurde durch eine Grignard-Reaktion von *rac.*-1-Dimethylamino-2-methyl-pentan-3-on mit 3-Methoxy-phenyl-magnesiumbromid in THF eine Mischung der vier Stereoisomere von 1-Dimethylamino-3-(3-methoxyphenyl)-2-methylpentan-3-ol hergestellt.

Die beiden Enantiomere (2R,3R)- und (2S,3S)-1-Dimethytamino-3-(3-methoxyphenyl)-2-methylpentan-3-ol und die beiden Enantiomere (2R,3S)- und (2S,3R)-1-Dimethylamino-3-(3-methoxyphenyl)-2-methylpentan-3-ol lagen jeweils in gleicher Menge, d.h. als Racemat vor. Das Verhältnis der Diastereomere (2R,3R)/(2S,RS) zu (2R,3S)/(2S, 3R) betrug 70 Gew.-% zu 30 Gew.-%.

__Beispiel 2__

Diastereomerentrennung [Trennung des Enantiomerenpaars der Konfiguration (2R,3R)/(2S,3S) vom Enantiomerenpaar der Konfiguration (2R,3S)/(2S,3R)]

a) HCl-Fällung

In einer 100 l Doppelmantelreaktionsanlage mit elektrischem Impellerrüher, Gasüberleitungsrohr, Pt100 Temperaturmesseinrichtung und ölbasierendem Kühl/Heizsystem wurden 15 kg (59,7 mol) einer Mischung des Enantiomerenpaars (2R,3R)/(2S,3S)-1-Dimethylamino-3-(3-methoxyphenyl)-2-methylpentan-3-ol (70%) und des Enantiomerenpaars (2R,3S)/(2S,3R)-1-Dimethylamino-3-(3-methoxyphenyl)-2-methylpentan-3-ol (30%) bei 20°C und einer Rührdrehzahl von 100 U/min in 70 l Aceton vorgelegt. Die Lösung wurde auf 5°C abgekühlt. Innerhalb von 20 min wurde ca. 2,0 kg gasförmiger Chlorwasserstoff über die Lösung geleitet, wobei die Temperatur im Kessel trotz Kühlung kontinuierlich auf 28°C anstieg. Der Chlorwasserstoff wurde aus einer Chlorwasserstoffflasche entnommen, die über einen Druckminderer und Schlauch mit dem Überleitungsrohr des Kessels verbunden war und zur Massenmessung auf einer Waage stand. Dann wurde noch soviel Chlowasserstoffgas übergeleitet, bis eine 5 ml Probe der Lösung, verdünnt mit 10 ml Wasser, einen pH-Wert von 1-3 hatte. Die Lösung wurde bei 5°C für 2 h gerührt. Die Suspension wurde über eine Zentrifuge abzentrifugiert und einmal mit 10 l Aceton nachgewaschen. Das Produkt wurde im Trockenschrank bei 40°C für 24 h im Vakuum bis zu einem Endruck von 20 mbar getrocknet. Man erhielt 11,16 kg (65% der Theorie) des Enantiomerenpaars (2R,3R)/(2S,3S)-1-Dimethylamino-3-(3-methoxyphenyl)-2-methylpentan-3-ol hydrochlorid als farbloses Produkt. Der Restgehalt des nicht gewünschten Enantiomerenpaars (2S,3R)/(2R,3S)-1-Dimethylamino-3-(3-methoxyphenyl)-2-methylpentan-3-ol hydrochlorid betrug 3%.

b) Überführen des Hydrochlorids in die freie Base

In einer 100 l Doppelmantelreaktionsanlage mit elektrischem Impellerrüher, Pt100 Temperaturmesseinrichtung und ölbasierendem Kühl/Heizsystem wurden die 11,16 kg (38,78 mol) des Enantiomerenpaars (2R,3R)/(2S,3S)-1-Dimethylamino-3-(3-methoxyphenyl)-2-methylpentan-3-ol hydrochlorid aus Schritt a), verunreinigt mit 3% des Enantiomerenpaars (2R,3S)/(2S,3R)-1-Dimethylamino-3-(3-methoxyphenyl)-2-methylpentan-3-ol hydrochlorid bei 20°C in 21 l Wasser vorgelegt. Die Lösung wurde bis zu einem pH-Wert von 13 mit 32 Gew.-%iger wässriger Natronlauge versetzt und anschließend mit 21 l Essigsäureethylester extrahiert. Nach Eindampfen des Essigsäureethylesters bei 60-65°C bis zu einem Endvakuum von 20 mbar verblieb die Base als farbloses Öl mit einer Ausbeute von 8,77 kg (90% der Theorie), verunreinigt mit 3% des Enantiomerenpaars (2S,3R)/(2R,3S)-1-Dimethylamino-3-(3-methoxyphenyl)-2-methylpentan-3-ol.

c) Umkristallisation als Hydrochlorid

In einer 100 l Doppelmantelreaktionsanlage mit elektrischem Impellerrüher, Gasüberleitungsrohr, Pt100 Temperaturmesseinrichtung und ölbasierendem Kühl/Heizsystem wurden 15 kg (59,7 mol) einer Mischung des Enantiomerenpaars (2R,3R)/(2S,3S)-1-Dimethylamino-3-(3-methoxyphenyl)-2-methylpentan-3-ol (97%) und des Enantiomerenpaars (2R,3S)/(2S,3R)-1-Dimethylamino-3-(3-methoxyphenyl)-2-methylpentan-3-ol (3%) bei 20°C und einer Rührdrehzahl von 100 U/min in 70 l Aceton vorgelegt. Die Lösung wurde auf 5°C abgekühlt. Innerhalb von 20 min wurde ca. 2,0 kg gasförmiger Chlorwasserstoff über die Lösung geleitet, wobei die Temperatur im Kessel trotz Kühlung kontinuierlich auf 28°C anstieg. Der Chlorwasserstoff wurde aus einer Chlorwasserstoffflasche entnommen, die über einen Druckminderer und Schlauch mit dem Überleitungsrohr des Kessels verbunden war und zur Massenmessung auf einer Waage stand. Dann wurde noch soviel Chlowasserstoffgas übergeleitet bis eine 5 ml Probe der Lösung, verdünnt mit 10 ml Wasser, einen pH-Wert von1-3 hatte. Die Lösung wurde bei 5°C für 2 h gerührt. Die Suspension wurde über eine Zentrifuge abzentrifugiert und einmal mit 10 l Aceton nachgewaschen. Das Produkt wurde im Trockenschrank bei 40°C für 24 h im Vakuum bis zu einem Endruck von 20mbar getrocknet. Man erhielt 13,74 kg (80% der Theorie) des farblosen Enantiomerenpaars (2R,3R)/(2S,3S)-1-Dimethylamino-3-(3-methoxyphenyl)-2-methylpentan-3-ol hydrochlorid mit einer Diastereomerenreinheit von 100%.

d) Überführen des Hydrochlorids in die freie Base

In einer 100 l Doppelmantelreaktionsanlage mit elektrischem Impellerrüher, Pt100 Temperaturmesseinrichtung und ölbasierendem Kühl/Heizsystem wurden 13,74 kg (47,7 mol) des Enantiomerenpaars (2R,3R)/(2S,3S)-1-Dimethylamino-3-(3-methoxyphenyl)-2-methylpentan-3-ol hydrochlorid bei 20°C in 26 l Wasser vorgelegt. Die Lösung wurde bis zu einem pH-Wert von 13 mit 32 Gew.-%iger wässriger Natronlauge versetzt und anschließend mit 26 l Essigsäureethylester extrahiert. Nach Eindampfen des Essigesters bei 60-65°C bis zu einem Endvakuum von 20 mbar verblieb das Enantiomerenpaar (2R,3R)/(2S,3S)-1-Dimethylamino-3-(3-methoxyphenyl)-2-methylpentan-3-ol als farbloses Öl mit einer Ausbeute von 10,80 kg (90% der Theorie) und einer Diastereomerenreinheit von 100%.

**Beispiel 3**

Trennung der beiden Enantiomere der Konfiguration (2R,3R) und (2S,3S) mit Hilfe von (+)-Di-O,O'-p-toluyl-weinsäure

a) Präzipitation von (2S,3S)-1-Dimethylamino-3-(3-methoxy-phenyl)-2-methyl-pentan-3-ol, (+)-di-O,O'-p-toluyl-tartrat

In einem 2 l Dreihalskolben mit Thermometer, mechanischem Luftdruckrührer, Rückflusskühler und Ölbadheizung wurden 121 g (0,48 mol) des Enantiomerenpaars (2R,3R)/(2S,3S)-1-Dimethylamino-3-(3-methoxyphenyl)-2-methylpentan-3-ol (mit einer Diastereomerenreinheit von 100%) in 100 ml 2-Butanon vorgelegt und unter Rühren mit einer Lösung von 185,6 g (0,48 mol) (+)-Di-O,O'-p-toluyl-weinsäure gelöst in 1700 ml 2-Butanon versetzt. Nach 48 h bei Raumtemperatur wurde der entstandene Kristallbrei über eine Nutsche im Vakuum abgesaugt und zweimal mit je 150 ml gekühltem (3-8°C) 2-Butanon gewaschen. Das Produkt wurde 24 h bei einer Temperatur von 40°C im Trockenschrank und einem Endvakuum von 20 mbar getrocknet. Man erhielt 125 g (40% der Theorie) (2S,3S)-1-Dimethylamino-3-(3-methoxy-phenyl)-2-methyl-pentan-3-ol (+)-di-O,O'-p-toluyl-tartrat, welches ohne weitere Reinigung zur Basenfreisetzung eingesetzt wurde.

b) Überführen des (+)-Di-O,O'-p-toluyl-tartrats in die freie Base

125 g (0,2 mol) (2S,3S)-1-Dimethylamino-3-(3-methoxy-phenyl)-2-methyl-pentan-3-ol (+)-di-O,O'-p-toluyl-tartrat wurden in 500ml Wasser gelöst, unter Rühren mit 18ml 37 Gew.%iger Salzsäure versetzt und zweimal mit je 150 ml Diethylether extrahiert. Dann wurde zur Wasserphase 35 ml 32 Gew.-%iger wässriger Natronlauge gegeben und anschließend mit 2 x 250ml Dichlormethan extrahiert. Nach Abdestillation des Lösungsmittels bei 40°C bis zu einem Enddruck von 20 mbar wurden 47 g (95% der Theorie ) eines farblosen Öls erhalten, das aus 83% (2S,3S)-1-Dimethylamino-3-(3-methoxy-phenyl)-2-methyl-pentan-3-ol und 17% (2R,3R)-1-Dimethylamino-3-(3-methoxy-phenyl)-2-methylpentan-3-ol bestand.

c) Isolation des anderen Enantiomers aus der Mutterlauge als Hydrochlorid und Überführung in die freie Base

Die Mutterlauge der Fällung aus Schritt a) wurde mit den beiden Waschlösungen vereinigt, mit 820 ml Wasser versetzt und unter Rühren 30 ml 37 Gew.-%iger Salzsäure zugegeben. Die wässrige Phase wurde zweimal mit je 250 ml Diethylether extrahiert. Zur Freisetzung der Base wurde die wässrige Phase mit 57 ml 32 Gew.-%iger wässriger Natronlauge versetzt und anschließend mit 2 x 250ml Dichlormethan extrahiert. Nach Abdestillation des Lösungsmittels bei 40°C bis zu einem Enddruck von 20 mbar wurden 70 g (58% der Theorie ) eines farblosen Öls erhalten, das aus 70% (2R,3R)-1-Dimethylamino-3-(3-methoxy-phenyl)-2-methyl-pentan-3-ol und 30% (2S,3S)-1-Dimethylamino-3-(3-methoxy-phenyl)-2-methyl-pentan-3-ol bestand.

**Beispiel 4**

Trennung der beiden Enantiomere der Konfiguration (2R,3R) und (2S,3S) mit Hilfe von (-)-Di-O,O'-p-toluyl-weinsäure

a) Präzipitation von (2R,3R)-1-Dimethylamino-3-(3-methoxy-phenyl)-2-methylpentan-3-ol (-)-di-O,O'-p-toluyl-tartrat

13,7 g (54,5 mmol) des Enantiomerenpaars (2R,3R)/(2S,3S)-1-Dimethylamino-3-(3-methoxyphenyl)-2-methylpentan-3-ol (mit einer Diastereomerenreinheit von 100%) wurden in 10 ml 2-Butanon vorgelegt und unter Rühren mit einer Lösung von 22 g (56,9 mmol) (-)-Di-O,O'-p-toluyl-weinsäure in 220ml 2-Butanon versetzt. Nach 24 h bei Raumtemperatur wurde der entstandene Kristallbrei im Vakuum über eine Nutsche abgesaugt und zweimal mit je 15 ml gekühltem (3-8°C) 2-Butanon gewaschen. Das Produkt wurde 24 h im Trockenschrank bei einer Temperatur von 40°C und einem Endvakuum von 20 mbar getrocknet. Man erhielt 8,7 g (25% der Theorie) (2R,3R)-1-Dimethylamino-3-(3-methoxy-phenyl)-2-methyl-pentan-3-ol (-)-di-O,O'-p-toluyl-tartrat, welches ohne weitere Reinigung zur Basenfreisetzung eingesetzt wurde.

b) Überführen des (-)-Di-O,O'-p-toluyl-tartrats in die freie Base

In einem 100ml Dreihalskolben mit Thermometer, magnetischem Rührer und Rückflusskühler wurden 8,7 g (13,6 mmol) (2R,3R)-1-Dimethylamino-3-(3-methoxyphenyl)-2-methylpentan-3-ol (-)-di-O,O'-p-toluyl-tartrat in 30 ml Wasser gelöst, unter Rühren mit 1,1 ml 37 Gew.-%iger Salzsäure versetzt und zweimal mit je 15ml Diethylether extrahiert. Dann wurde zur Wasserphase 2,2 ml 32 Gew.-%iger wässriger Natronlauge gegeben und anschließend mit 2 x 20 ml Dichlormethan extrahiert. Nach Abdestillation des Lösungsmittels bei 40°C bis zu einem Enddruck von 20 mbar wurden

3,36 g (98% der Theorie ) eines farblosen Öls erhalten, das aus 65% (2R,3R)-1-Dimethylamino-3-(3-methoxy-phenyl)-2-methyl-pentan-3-ol und 35% (2S,3S)-1-Dimethylamino-3-(3-methoxy-phenyl)-2-methyl-pentan-3-ol bestand.

**Vergleichsbeispiel 5**

Trennung der beiden Enantiomere der Konfiguration (2R,3R) und (2S,3S) mit Hilfe von D-(-)-weinsäure nach Anreicherung des Enantiomers mit Konfiguration (2R,3R)

a) Präzipitation von (2R,3R)-1-Dimethylamino-3-(3-methoxy-phenyl)-2-methyl-pentan-3-ol (-)-tartrat

30 g (0,12 mol) des in Beispiel 4b) erhaltenen Gemisches der beiden Enantiomere mit einem Enantlomerenüberschuss von 30%ee (65 Gew.-% (2R,3R)-1-Dimethylamino-3-(3-methoxyphenyl)-2-methylpentan-3-ol und 35 Gew.-% (2S,3S)-1-Dimethylamino-3-(3-methoxyphenyl)-2-methylpentan-3-ol) wurden in einem 2 l Dreihalskolben mit Thermometer, mechanischem Luftdruckrührer, Rückflusskühler und Ölbadheizung in 50 ml Ethanol vorgelegt und unter Rühren mit einer Lösung von 18,0 g (0,12 mol) (-)-(2S,3S)-weinsäure in 200ml Ethanol versetzt. Nach 1 h wurde bei Raumtemperatur 200ml Diethylether zugegeben und 24 h nachgerührt. Der entstandene Kristallbrei wurde im Vakuum über eine Nutsche abgesaugt und zweimal mit je 200 ml gekühltem (3-8°C) Diethylether gewaschen. Das Produkt wurde 24 h im Trockenschrank bei einer Temperatur von 40°C und einem Endvakuum von 20 mbar getrocknet. Man erhielt 26,4 g (55% der Theorie) (2R,3R)-1-Dimethylamino-3-(3-methoxy-phenyl)-2-methyl-pentan-3-ol (-)-tartrat, das ohne weitere Reinigung zur Basenfreisetzung eingesetzt wurde.

b) Überführung des (-)-Tartrats in die freie Base

26,4 g (65,8 mmol) (2R,3R)-1 -Dimethylamino-3-(3-methoxy-phenyl)-2-methyl-pentan-3-ol (-)-tartrat wurden in 100 ml Wasser gelöst, unter Rühren mit 7,4 ml 32 Gew.-%Iger wässriger Natronlauge versetzt und anschließend mit 2 x 50ml Dichlormethan extrahiert. Nach Abdestillation des Lösungsmittels bei 40°C bis zu einem Enddruck von 20 mbar wurden 16,2 g (98% der Theorie ) eines farblosen Öls erhalten, das aus 98% (2R,3R)-1-Dimethylamino-3-(3-methoxy-phenyl)-2-methyl-pentan-3-ol und 2% (2S,3S)-1-Dimethylamino-3-(3-methoxy-phenyl)-2-methyl-pentan-3-ol bestand.

**Vergleichsbeispiel 6**

Trennung der beiden Enantiomere der Konfiguration (2R,3R) und (2S,3S) mit Hilfe von L-(+)-weinsäure nach Anreicherung des Enantiomers mit Konfiguration (2S,3S)

a) Präzipitation von (2S,3S)-1-Dimethylamino-3-(3-methoxyphenyl)-2-methylpentan-3-ol (+)-tartrat

In einer 100 l Doppelmantelreaktionsanlage mit elektrischem Impellerrüher, Pt100 Temperaturmesseinrichtung und ölbasierendem Kühl/Heizsystem wurden 6,93 kg (46,17 mol) (+)-(2R,3R)-weinsäure in 75 l Ethanol vorgelegt. Dann wurden 10,55 kg (41,97 mol) einer racemischen Mischung des Enantiomerenpaars (2R,3R)/(2S,3S)-1-Dimethylamino-3-(3-methoxy-phenyl)-2-methyl-pentan-3-ol in 3,5 l Ethanol vorgelegt. Das racemische Gemisch wurde durch Zugabe von 1,06 kg (4,21 mol) der enantiomerenreinen Verbindung (2S,3S)-1-Dimethylamino-3-(3-methoxy-phenyl)-2-methyl-pentan-3-ol hinsichtlich des Enantiomers mit Konfiguration (2S,3S) angereichert. Diese Mischung wurde zu der vorgelegten Weinsäure bei 10°C und einer Rührdrehzahl von 150 U/min gegeben und 20 Stunden gerührt. Die Kristalle wurden abzentrifugiert und im Trockenschrank bei 50°C im Vakuum für 12 h getrocknet. Es wurden nach 24 h Trocknung im Trockenschrank bei 40°C und einem Enddruck von 20 mbar 8,34 kg (20,77 mol, 45% der Theorie) (2S,3S)-1-Dimethylamino-3-(3-methoxy-phenyl)-2-methyl-pentan-3-ol (+)-tartrat als farblose Kristalle isoliert, die so getrocknet oder ethanolfeucht zur Freisetzung der Base eingesetzt wurden. Aus der Mutterlauge der Zentrifugation konnte das andere Enantiomer (2R,3R)-1-Dimethylamino-3-(3-methoxy-phenyl)-2-methyl-pentan-3-ol gewonnen werden.

b) Überführung des (+)-Tartrats in die freie Base

In einer 100 l Doppelmantelreaktionsanlage mit elektrischem Impellerrüher, Pt100 Temperaturmesseinrichtung und ölbasierendem Kühl/Heizsystem wurden 16,68 kg (41,55 mol) ethanolfeuchtes oder getrocknetes (2S,3S)-1-Dimethylamino-3-(3-methoxy-phenyl)-2-methyl-pentan-3-ol (+)-tartrat aus Schritt a) in 65 l Wasser gelöst und mit ca. 5,2 kg 32 Gew.-%iger Natronlauge versetzt, bis ein pH-Wert von 12-13 erreicht wurde. Die Temperatur wurde durch Kühlung des Reaktionskessels unterhalb von 35°C gehalten. Es wurden 30 l Essigsäureethylester zugegeben und nach 10 min unter Rühren der Rührer zur Phasentrennung abgeschaltet. Die untere wässrige Phase wurde abgelassen und die obere organische Phase bei einer maximalen Innentemperatur von 50°C im Vakuum bis 10 mbar abdestilliert. Der verbleibende

hellgelbe ölige Rückstand war (2S,3S)-1-Dimethylamino-3-(3-methoxyphenyl)-2-methylpentan-3-ol hydrochlorid. Die Ausbeute betrug 9,92 kg (95% der Theorie) mit einer Enantiomerenreinheit von 98,5%.

c) Umkristallisation als Hydrochlorid

In einer 100 l Doppelmantelreaktionsanlage mit elektrischem Impellerrüher, Gasüberleitungsrohr, Pt100 Temperaturmesseinrichtung und ölbasierendem Kühl/Heizsystem wurden 15 kg (59,7 mol) einer Mischung aus (2S,3S)-1-Dimethylamino-3-(3-methoxyphenyl)-2-methylpentan-3-ol (98%) und (2R,3R)-1-Dimethylamino-3-(3-methoxyphenyl)-2-methylpentan-3-ol (2%) bei 20°C und einer Rührdrehzahl von 100 U/min in 70 l Aceton vorgelegt. Die Lösung wurde auf 5°C abgekühlt. Innerhalb von 20 min wurde ca. 2,0 kg gasförmiger Chlorwasserstoff über die Lösung geleitet, wobei die Temperatur im Kessel, trotz Kühlung, kontinuierlich auf 28°C anstieg. Der Chlorwasserstoff wird aus einer Chlorwasserstoffflasche entnommen, die über einen Druckminderer und Schlauch mit dem Überleitungsrohr des Kessels verbunden war und zur Massenmessung auf einer Waage stand. Dann wurde noch soviel Chlowasserstoffgas übergeleitet, bis eine 5 ml Probe der Lösung, verdünnt mit 10 ml Wasser, einen pH-Wert von1-3 hatte. Die Suspension wurde bei 5°C für 2 h gerührt. Anschließend wurde über eine Zentrifuge abzentrifugiert und mit 10 l Aceton nachgewaschen. Das Produkt wurde im Trockenschrank bei 40°C für 24 h im Vakuum bis zu einem Endruck von 20 mbar getrocknet. Man erhielt 13,74 kg (80% der Theorie) farbloses (2S,3S)-1-Dimethylamino-3-(3-methoxyphenyl)-2-methylpentan-3-ol hydrochlorid mit einer Enantiomerenreinheit von 100%.

d) Isolieren des anderen Enantiomers aus der Mutterlauge von Schritt a) als freie Base

In einer 100 l Doppelmantelreaktionsanlage mit elektrischem Impellerrüher, Pt100 Temperaturmesseinrichtung und ölbasierendem Kühl/Heizsystem wurde das Ethanol der Mutterlauge der Zentrifugation der Fällung von (2S,3S)-1-Dimethylamino-3-(3-methoxyphenyl)-2-methylpentan-3-ol (+)-tartrat bei einer Innentemperatur von 75°C und einem Enddruck von < 50 mbar abdestilliert. Der Rückstand wurde in 22 l Wasser gelöst und mit 32 Gew.-%iger Natronlauge versetzt, bis ein pH-Wert von 12-13 erreicht wurde. Die Innentemperatur wurde durch Kühlung des Reaktionskessels unterhalb von 25°C gehalten. Es wurden 22 l Essigsäureethylester zugegeben, 10 min gerührt und der Rührer zur Phasentrennung abgeschaltet. Die untere wässrige Phase wurde abgelassen und mit weiteren 11 l Essigsäureethylester extrahiert. Die beiden vereinigten Essigsäureethylesterphasen wurden bei einer maximalen Innentemperatur von 60°C im Vakuum bis zu einem Enddruck von 10 mbar abdestilliert. Die Ausbeute an (2R,3R)-1-Dimethylamino-3-(3-methoxyphenyl)-2-methylpentan-3-ol betrug 5,8 kg (50% der Theorie).

**Vergleichsbeispiel 7**

Isolierung des angereicherten Enantiomers der Konfiguration (2S,3S) mit Hilfe von L-(+)-weinsäure aus dem Gemisch aller vier Diastereomere [Produkt der Grignard-Reaktion in Beispiel 1]

In einer 100 l Doppelmantelreaktionsanlage mit elektrischem Impellerrüher, Pt100 Temperaturmesseinrichtung und ölbasierendem Kühl/Heizsystem wurden 6,93 kg (46,17 mol) (+)-(2R,3R)-weinsäure in 75 l Ethanol vorgelegt. Dann wurde eine Mischung aus 10,55 kg (41,97 mol) des Enantiomerenpaars (2R,3R)/(2S,3S)-1-Dimethylamino-3-(3-methoxy-phenyl)-2-methyl-pentan-3-ol (70%) und des Enantiomerenpaars (2R,3S)/(2S,3R)-1-Dimethylamino-3-(3-methoxy-phenyl)-2-methyl-pentan-3-ol (30%) in 3,5 l Ethanol durch Zugabe von 1,06 kg (4,21 mol) der enantiomerenreinen Verbindung (2S,3S)-1-Dimethylamino-3-(3-methoxy-phenyl)-2-methyl-pentan-3-ol hinsichtlich des Enantiomers mit Konfiguration (2S,3S) angereichert. Diese Mischung wurde zu der vorgelegten Weinsäure bei 10°C und einer Rührdrehzahl von 150 U/min gegeben und 20 Stunden gerührt. Die Kristalle wurden abzentrifugiert und im Trockenschrank bei 50°C für 12 h im Vakuum bis zu einem Enddruck von 20 mbar getrocknet. Es wurden 5,93 kg (32% der Theorie) farblose Kristalle von 1-Dimethylamino-3-(3-methoxy-phenyl)-2-methyl-pentan-3-ol (+)-tartrat mit einer Zusammensetzung der Stereoisomere von 98,2% (2S,3S)-1-Dimethylamino-3-(3-methoxyphenyl)-2-methylpentan-3-ol (+)-tartrat, 0,8% (2R, 3R)-1-Dimethylamino-3-(3-methoxyphenyl)-2-methylpentan-3-ol (+)-tartrat und 1 % des Enantiomerenpaars (2R,3S)/(2S,3R)-1-Dimethylamino-3-(3-methoxyphenyl)-2-methylpentan-3-ol (+)-tartrat erhalten.

**Beispiel** 8

Trennung der beiden Enantiomere der Konfiguration (2R,3R) und (2S,3S) mit Hilfe von Phosphorsäure-Präzipitation des Enantiomers der Konfiguration (2S,3S)

a) mit Animpfen

10 g (0,04 mol) des Enantiomerenpaars (2R,3R)/(2S,3S)-1-Dimethylamino-3-(3-methoxy-phenyl)-2-methyl-pentan-3-ol wurden in 40ml Ethanol gelöst, unter Rühren mit 3 g 85 Gew.-%iger ortho-Phosphorsäure versetzt und mit 1 g (2,86 mmol) (2S,3S)-1-Dimethylamino-3-(3-methoxy-phenyl)-2-methyl-pentan-3-ol phosphat angeimpft. Nach 24 h wurden die gebildeten Kristalle abgesaugt und für 24 h im Trockenschrank bei 40°C bis zu einem Enddruck von 10 mbar getrocknet. Man erhielt 3,3 g (22% der Theorie) (2S,3S)-1-Dimethylamino-3-(3-methoxyphenyl)-2-methylpentan-3-ol phosphat als farblose Kristalle mit einer Enantiomerenreinheit von 99,1%.

b) ohne Animpfen

10 g (0,04 mol) des Enantiomerenpaars (2R,3R)/(2S,3S)-1-Dimethylamino-3-(3-methoxy-phenyl)-2-methyl-pentan-3-ol wurden in 40 ml Ethanol gelöst und unter Rühren mit 3 g 85 Gew.-%iger ortho-Phosphorsäure versetzt. Nach 24 h wurden die gebildeten Kristalle abgesaugt und 24 h im Trockenschrank bei 40°C bis zu einem Enddruck von 10 mbar getrocknet. Man erhielt 2,2 g (16% der Theorie) (2S,3S)-1-Dimethylamino-3-(3-methoxyphenyl)-2-methylpentan-3-ol phosphat als farblose Kristalle mit einer Enantiomerenreinheit von 85%.

**Beispiel 9**

Trennung der beiden Enantiomere der Konfiguration (2R,3R) und (2S,3S) mit Hilfe von Phosphorsäure-Präzipitation des Enantiomers der Konfiguration (2R,3R) aus der Mutterlauge der (+)-weinsäurefällung von Beispiel 6

a) 1.Phosphatfällung und Basenfreisetzung

In einer 100 l Doppelmantelreaktionsanlage mit elektrischem Impellerrüher, Pt100 Temperaturmesseinrichtung und ölbasierendem Kühl/Heizsystem wurden die 7 kg (27,84 mol) (2R,3R)-1-Dimethylamino-3-(3-methoxyphenyl)-2-methylpentan-3-ol aus Beispiel 6 d) in 42 l Ethanol bei einer Temperatur von 25°C gelöst. Bei einer Rührgeschwindigkeit von 100 $min^{-1}$ wurden 2,16 kg 85 Gew.-%ige ortho-Phosphorsäure zugegeben und die Lösung auf 5°C gekühlt. Nach 24 h wurden die gebildeten Kristalle abzentrifugiert und mit 7 l Ethanol nachgewaschen. Der Niederschlag (Phosphatsalz) wurde in 42 l Wasser gelöst und mit 32 Gew.-%iger Natronlauge versetzt, bis ein pH-Wert von 12-13 erreicht wurde. Die Temperatur wurde durch Kühlung des Reaktionskessels unterhalb von 35°C gehalten. Es wurden 20 l Essigsäureethylester zugegeben und nach 10 min unter Rühren der Rührer zur Phasentrennung abgeschaltet. Die untere wässrige Phase wurde erneut mit 12 l Essigsäureethylester extrahiert, die organischen Phasen vereinigt und bei einer maximalen Innentemperatur von 50°C im Vakuum bis 10 mbar abdestilliert. Es wurden 4.9 kg (70% der Theorie) eines farblosen Öls erhalten, das so in die zweite Phoshatfällung eingesetzt wurde.

b) 2.Phosphatfällung und Basenfreisetzung

In einer 100 l Doppelmantelreaktionsanlage mit elektrischem Impellerrüher, Pt100 Temperaturmesseinrichtung und ölbasierendem Kühl/Heizsystem wurden 4,9 kg (19,5 mol) des (2R,3R)-1-Dimethylamino-3-(3-methoxyphenyl)-2-methylpentan-3-ol aus Schritt a) in 30 l Ethanol bei einer Temperatur von 25°C gelöst. Bei einer Rührgeschwindigkeit von 100 $min^{-1}$ wurden 1,51 kg 85 Gew.-%ige ortho- Phosphorsäure zugegeben und die Lösung auf 5°C gekühlt. Nach 24 h wurden die gebildeten Kristalle abzentrifugiert und mit 5 l Ethanol nachgewaschen. Der Niederschlag wurde in 30 l Wasser gelöst und mit 32 Gew.-%iger Natronlauge versetzt, bis ein pH-Wert von 12-13 erreicht wurde. Die Temperatur wurde durch Kühlung des Reaktionskessels unterhalb von 35°C gehalten. Es wurden 14 l Essigsäureethylester zugegeben und nach 10 min unter Rühren der Rührer zur Phasentrennung abgeschaltet. Die untere wässrige Phase wurde erneut mit 9 l Essigsäureethylester extrahiert, die organischen Phasen vereinigt und bei einer maximalen Innentemperatur von 50°C im Vakuum bis 10 mbar abdestilliert. Es wurden 2,5 kg (51% der Theorie) eines farblosen Öls mit einer Enantiomerenreinheit von 97% erhalten.

c) Umkristallisation als Hydrochlorid

In einer 100 l Doppelmantelreaktionsanlage mit elektrischem Impellerrüher, Gasüberleitungsrohr, Pt100 Temperaturmesseinrichtung und ölbasierendem KÜhl/Heizsystem wurden die 10 kg (39,8 mol) der Mischung aus (2R,3R)-1-Dimethylamino-3-(3-methoxyphenyl)-2-methylpentan-3-ol (97%) und (2S,3S)-1-Dimethylamino-3-(3-methoxyphenyl)-2-methylpentan-3-ol (3%) aus Schritt b) bei 20°C und einer Rührdrehzahl von 100 U/min in 45 l Aceton vorgelegt. Die Lösung wurde auf 5°C abgekühlt. Innerhalb von 20 min wurde ca. 1,3 kg gasförmiger Chlorwasserstoff über die Lösung geleitet, wobei die Temperatur im Kessel trotz Kühlung kontinuierlich auf 28°C anstieg. Der Chlorwasserstoff wird aus einer Chlorwasserstoffflasche entnommen, die über einen Druckminderer und Schlauch mit dem Überleitungsrohr des Kessels verbunden war und zur Massenmessung auf einer Waage stand. Dann wurde noch soviel Chlorwasserstoffgas übergeleitet bis eine 5ml Probe der Lösung, verdünnt mit 10 ml Wasser, einen pH-Wert von1-3 hatte. Die Lösung wurde bei 5°C für 2h gerührt. Die Suspension wurde über eine Zentrifuge abzentrifugiert und einmal mit 7 l Aceton nachgewaschen. Das Produkt wurde im Trockenschrank bei 40°C für 24h im Vakuum bis zu einem Endruck von 20mbar getrocknet. Man erhielt 9,73 kg (85% der Theorie) farbloses (2R,3R)-1-Dimethylamino-3-(3-methoxyphenyl)-2-methylpentan-3-ol hydrochlorid mit einer Enantiomerenreinheit von 100%.

## Patentansprüche

1. Verfahren zur Isolierung eines Stereoisomers aus einer Mischung umfassend die beiden Stereoisomere der allgemeinen Formeln (I-A) und (I-A') und/oder die beiden Stereoisomere der allgemeinen Formeln (I-B) und (I-B')

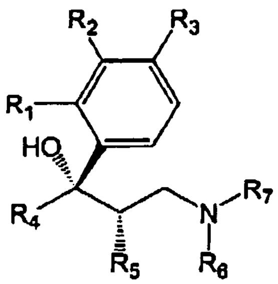
(I-A)

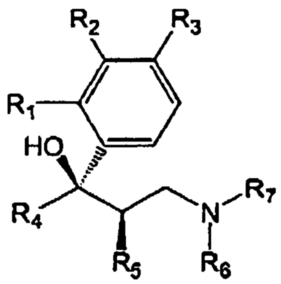
(I-A')

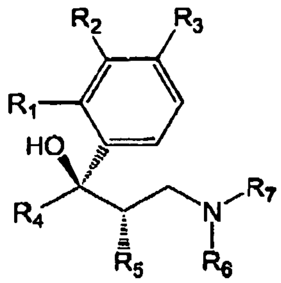
(I-B)

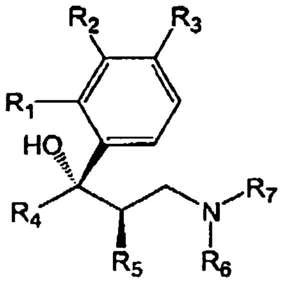
(I-B')

worin
$R_1$, $R_2$ und $R_3$ gleich oder verschieden ausgewählt sind aus der Gruppe bestehend aus -H, -F, -Cl, -$C_1$-$C_6$-alkyl, -S-$C_1$-$C_6$-alkyl, -OH, -O-$C_1$-$C_6$-alkyl, -O-$C_1$-$C_6$-alkylen-phenyl, -OCO-$C_1$-$C_6$-alkyl, -$OCO_2$-$C_1$-$C_6$-alkyl, -OCON($C_1$-$C_6$-alkyl)$_2$ und -O-Si$R_8R_9R_{10}$ (worin $R_8$, $R_9$ und $R_{10}$ gleich oder verschieden -$C_1$-$C_6$-alkyl oder -Phenyl sind);
$R_4$ -H oder -$C_1$-$C_6$-alkyl ist;
$R_5$ -$C_1$-$C_6$-alkyl ist; und
$R_6$ und $R_7$ gleich oder verschieden -H oder -$C_1$-$C_6$-alkyl sind;
wobei
"$C_1$-$C_6$-alkyl" einen linearen oder verzweigten, cyclischen oder offenkettigen, gesättigten Kohlenwasserstoffrest mit 1 bis 6 Kohlenstoffatomen, ggf. mit 1 bis 6 Halogenatomen, gleich oder verschieden ausgewählt aus -F, -Cl und -Br, substituiert, bedeutet, und "$C_1$-$C_6$-alkylen" einen linearen oder verzweigten, cyclischen oder offenkettigen, gesättigten Kohlenwasserstoffrest mit 1 bis 6 Kohlenstoffatomen, ggf. mit 1 bis 6 Halogenatomen, gleich oder verschieden ausgewählt aus -F, -Cl und -Br, substituiert, bedeutet;
oder deren Salze mit organischen oder anorganischen Säuren;
umfassend die Schritte

(a) Beeinflussen des Mischungsverhältnisses der Stereoisomere in der Mischung, so dass zumindest eines der Stereoisomere mit einem Enantiomerenüberschuss vorliegt, und
(b) Zugeben von Phosphorsäure unter Bedingungen, welche die Präzipitation des im Enantiomerenüberschuss vorliegenden Stereoisomers aus einer Lösung als Phosphat, Hydrogenphosphat oder Dihydrogenphosphat bewirken.

2. Verfahren nach Anspruch 1, **dadurch gekennzeichnet, dass** die Beeinflussung in Schritt (a) erfolgt

- durch Zugabe des Stereoisomers, welches im Überschuss vorliegen soll, oder
- durch enantioselektive Synthese der Stereoisomere.

**3.** Verfahren nach Anspruch 1 oder 2, **dadurch gekennzeichnet, dass** der Enantiomerenüberschuss wenigstens 1,0%ee beträgt.

**4.** Verfahren nach einem der vorstehenden Ansprüche, **dadurch gekennzeichnet, dass** $R_1$ und $R_3$ jeweils -H sind und $R_2$ $-OCH_3$ ist.

**5.** Verfahren nach einem der vorstehenden Ansprüche, **dadurch gekennzeichnet, dass** $R_4$ $-CH_2CH_3$ ist und $R_5$, $R_6$ und $R_7$ jeweils $-CH_3$ sind.

**6.** Verfahren nach einem der vorstehenden Ansprüche, **dadurch gekennzeichnet, dass** es den Schritt umfasst

(c) Abtrennen des in Schritt (b) erhaltenen präzipitierten Salzes von der überstehenden Lösung.

**7.** Verfahren nach Anspruch 6, **dadurch gekennzeichnet, dass** es den Schritt umfasst

(d) Überführen des in Schritt (c) abgetrennten Salzes in das Hydrochlorid.

**8.** Additionssalz eines Stereoisomers der allgemeinen Formel (I-A), (I-A'), (I-B) oder (I-B') definiert wie in Anspruch 1, 4 oder 5 und Phosphorsäure.

**9.** Additionssalz nach Anspruch 8 ausgewählt aus der Gruppe bestehend aus

- (2R,3R)-1-Dimethylamlno-3-(3-methoxy-phenyl)-2-methyl-pentan-3-ol-phosphat,
- (2R,3R)-1-Dimethylamino-3-(3-methoxy-phenyl)-2-methyl-pentan-3-ol-hydrogenphosphat,
- (2R,3R)-1-Dimethylamino-3-(3-methoxy-phenyl)-2-methyl-pentan-3-ol-dihydrogenphosphat,
- (2S,3S)-1-Dimethylamino-3-(3-methoxy-phenyl)-2-methyl-pentan-3-ol-phosphat,
- (2S,3S)-1-Dimethylamino-3-(3-methoxy-phenyl)-2-methyl-pentan-3-ol-hydrogenphosphat,
- (2S,3S)-1-Dimethylamino-3-(3-methoxy-phenyl)-2-methyl-pentan-3-ol-dihydrogenphosphat,
- (2R,3S)-1-Dimethylamino-3-(3-methoxy-phenyl)-2-methyl-pentan-3-ol-phosphat,
- (2R,3S)-1-Dimethylamino-3-(3-methoxy-phenyl)-2-methyl-pentan-3-ol-hydrogenphosphat,
- (2R,3S)-1-Dimethylamino-3-(3-methoxy-phenyl)-2-methyl-pentan-3-ol-dihydrogenphosphat,
- (2S,3R)-1-Dimethylamino-3-(3-methoxy-phenyl)-2-methyl-pentan-3-ol-phosphat,
- (2S,3R)-1-Dimethylamino-3-(3-methoxy-phenyl)-2-methyl-pentan-3-ol-hydrogenphosphat und
- (2S,3R)-1-Dimethylamino-3-(3-methoxy-phenyl)-2-methyl-pentan-3-ol-dihydrogenphosphat.

**10.** Verfahren zur Herstellung eines Stereoisomers der allgemeinen Formel (I-A), (I-A'), (I-B) oder (I-B')

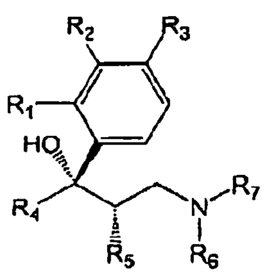
(I-A)

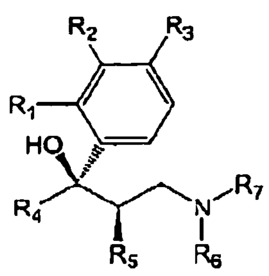
(I-A')

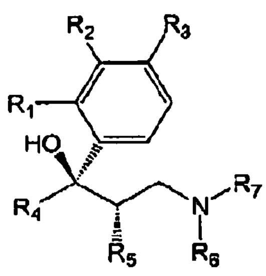
(I-B)

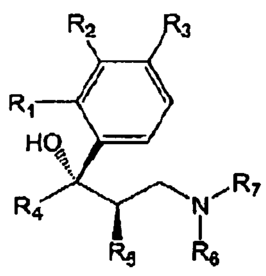
(I-B')

worin
$R_1$, $R_2$ und $R_3$ gleich oder verschieden ausgewählt sind aus der Gruppe bestehend aus -H, -F, -Cl, $-C_1-C_6$-alkyl, $-S-C_1-C_6$-alkyl und -OH, mit der Maßgabe, dass zumindest einer der Reste $R_1$, $R_2$ und $R_3$ -OH ist;
$R_4$ -H oder $-C_1-C_6$-alkyl ist;
$R_5$ $-C_1-C_6$-alkyl ist; und
$R_6$ und $R_7$ gleich oder verschieden -H oder $-C_1-C_6$-alkyl sind;
oder deren Salze mit organischen oder anorganischen Säuren;
umfassend das Verfahren gemäß einem der Ansprüche 1 bis 9, worin zumindest einer der Reste $R_1$, $R_2$ und $R_3$ -O-

$C_1$-$C_6$-alkyl, -O-$C_1$-$C_6$-alkylen-phenyl, -OCO-$C_1$-$C_6$-alkyl, -$OCO_2$-$C_1$-$C_6$-alkyl, -OCON($C_1$-$C_6$-alkyl)$_2$ oder -O-$SiR_8R_9R_{10}$ ist;
wobei
"$C_1$-$C_6$-alkyl" einen linearen oder verzweigten, cyclischen oder offenkettigen, gesättigten Kohlenwasserstoffrest mit 1 bis 6 Kohlenstoffatomen, ggf. mit 1 bis 6 Halogenatomen, gleich oder verschieden ausgewählt aus -F, -Cl und -Br, substituiert, bedeutet, und
"$C_1$-$C_6$-alkylen" einen linearen oder verzweigten, cyclischen oder offenkettigen, gesättigten Kohlenwasserstoffrest mit 1 bis 6 Kohlenstoffatomen, ggf. mit 1 bis 6 Halogenatomen, gleich oder verschieden ausgewählt aus -F, -Cl und -Br, substituiert, bedeutet.

**11.** Verfahren nach Anspruch 10, **dadurch gekennzeichnet, dass** es den Schritt umfasst

(e) Überführen des zumindest einen der Reste $R_1$, $R_2$ und $R_3$, welcher -O-$C_1$-$C_6$-alkyl, -O-$C_1$-$C_6$-alkylen-phenyl, -CCO-$C_1$-$C_6$-alkyl, -$OCO_2$-$C_1$-$C_6$-alkyl, -OCO-N($C_1$-$C_6$-alkyl)$_2$ oder -O-$SiR_8R_9R_{10}$ ist, in eine -OH Gruppe.

## Claims

**1.** Method for the isolation of a stereoisomer from a mixture comprising the two stereoisomers of the general formulae (I-A) and (I-A') and/or the two stereoisomers of the general formulae (I-B) and (I-B')

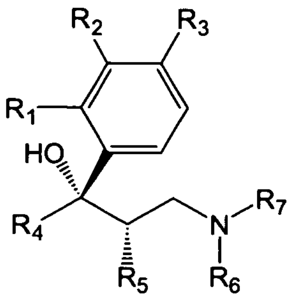
(I-A)

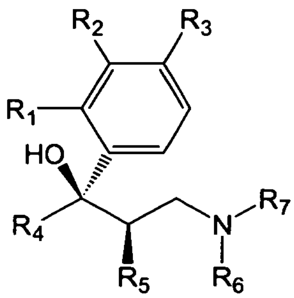
(I-A')

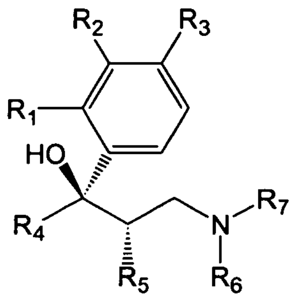
(I-B)

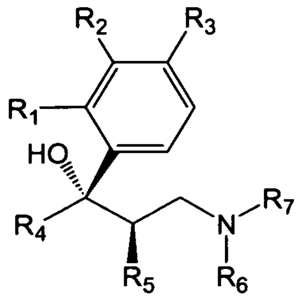
(I-B')

in which
$R_1$, $R_2$ and $R_3$, identical or different, are selected from the group consisting of -H, -F, -Cl, -$C_1$-$C_6$-alkyl, -S-$C_1$-$C_6$-alkyl, -OH, -O-$C_1$-$C_6$-alkyl, -O-$C_1$-$C_6$-alkylenephenyl, -OCO-$C_1$-$C_6$-alkyl, -$OCO_2$-$C_1$-$C_6$-alkyl, -OCON($C_1$-$C_6$-alkyl)$_2$ and -O-$SiR_8R_9R_{10}$ (in which $R_8$, $R_9$ and $R_{10}$, identical or different, are -$C_1$-$C_6$-alkyl or -phenyl);
$R_4$ is -H or -$C_1$-$C_6$-alkyl;
$R_5$ is -$C_1$-$C_6$-alkyl; and
$R_6$ and $R_7$, identical or different, are -H or -$C_1$-$C_6$-alkyl;
wherein
"$C_1$-$C_6$-alkyl" means a linear or branched, cyclic or open-chain saturated hydrocarbon residue with 1 to 6 carbon atoms, possibly substituted with 1 to 6 halogen atoms, identical or different, selected from F, Cl and Br, and "$C_1$-$C_6$-alkylene" means a linear or branched, cyclic or open-chain saturated hydrocarbon residue with 1 to 6 carbon atoms, possibly substituted with 1 to 6 halogen atoms, identical or different, selected from F, Cl and B;
or their salts with organic or inorganic acids;
comprising the steps

(a) manipulating the mixture ratio of the stereoisomers in the mixture so that at least one of the stereoisomers is present in an enantiomeric excess, and
(b) adding phosphoric acid under conditions which bring about precipitation of the stereoisomer present in enantiomeric excess from a solution as phosphate, hydrogenphosphate or dihydrogenphosphate.

**2.** Method according to claim 1, **characterised in that** the manipulation in step (a) is carried out

- by addition of the stereoisomer which is to be present in excess, or

- by enantioselective synthesis of the stereoisomers.

3. Method according to claim 1 or 2, **characterised in that** the enantiomeric excess is at least 1.0% ee.

4. Method according to one of the previous claims **characterised in that** $R_1$ and $R_3$ are in each case -H and $R_2$ is $-OCH_3$.

5. Method according to one of the previous claims, **characterised in that** $R_4$ is $-CH_2CH_3$ and $R_5$, $R_6$ and $R_7$ are in each case $-CH_3$.

6. Method according to one of the previous claims, **characterised in that** it comprises the step

(c) separating the precipitated salt obtained in step (b) from the supernatant solution.

7. Method according to claim 6, **characterised in that** it comprises the step (d) converting the salt separated in step (c) into the hydrochloride.

8. Addition salt of a stereoisomer of the general formula (I-A), (I-A'), (I-B) or (I-B') as defined in claim 1, 4 or 5 and phosphoric acid.

9. Addition salt according to claim 8 selected from the group consisting of

- (2R,3R)-1-dimethylamino-3-(3-methoxyphenyl)-2-methylpentan-3-ol phosphate,
- (2R,3R)-1-dimethylamino-3-(3-methoxyphenyl)-2-methylpentan-3-ol hydrogenphosphate,
- (2R,3R)-1-dimethylamino-3-(3-methoxyphenyl)-2-methylpentan-3-ol dihydrogenphosphate,
- (2S,3S)-1-dimethylamino-3-(3-methoxyphenyl)-2-methylpentan-3-ol phosphate,
- (2S,3S)-1-dimethylamino-3-(3-methoxyphenyl)-2-methylpentan-3-ol hydrogenphosphate,
- (2S,3S)-1-dimethylamino-3-(3-methoxyphenyl)-2-methylpentan-3-ol dihydrogenphosphate,
- (2R,3S)-1-dimethylamino-3-(3-methoxyphenyl)-2-methylpentan-3-ol phosphate,
- (2R,3S)-1-dimethylamino-3-(3-methoxyphenyl)-2-methylpentan-3-ol hydrogenphosphate,
- (2R,3S)-1-dimethylamino-3-(3-methoxyphenyl)-2-methylpentan-3-ol dihydrogenphosphate,
- (2S,3R)-1-dimethylamino-3-(3-methoxyphenyl)-2-methylpentan-3-ol phosphate,
- (2S,3R)-1-dimethylamino-3-(3-methoxyphenyl)-2-methylpentan-3-ol hydrogenphosphate and
- (2S,3R)-l-dimethylamino-3-(3-methoxyphenyl)-2-methylpentan-3-ol dihydrogenphosphate.

10. Method for the preparation of a stereoisomer of the general formula (I-A), (I-A'), (I-B) or (I-B')

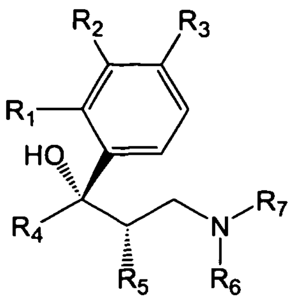
(I-A)

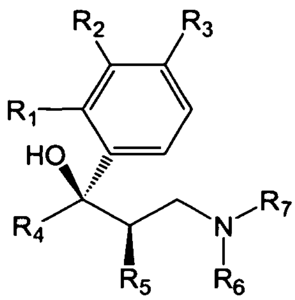
(I-A')

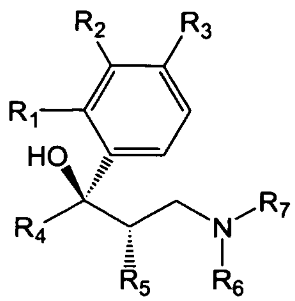
(I-B)

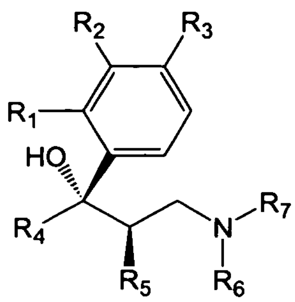
(I-B')

in which
$R_1$, $R_2$ and $R_3$, identical or different, are selected from the group consisting of -H, -F, -Cl, $-C_1-C_6$-alkyl, $-S-C_1-C_6$-alkyl and -OH, providing that at least one of the residues $R_1$, $R_2$ and $R_3$ is -OH;
$R_4$ is -H or $-C_1-C_6$-alkyl;
$R_5$ is $-C_1-C_6$-alkyl; and
$R_6$ and $R_7$, identical or different, are -H or $-C_1-C_6$-alkyl;
or their salts with organic or inorganic acids;
comprising the method according to one of claims 1 to 9, in which at least one of the residues $R_1$, $R_2$ and $R_3$ is -O-

$C_1$-$C_6$-alkyl, -O-$C_1$-$C_6$-alkylenephenyl, -OCO-$C_1$-$C_6$-alkyl, -$OCO_2$-$C_1$-$C_6$-alkyl, -OCON($C_1$-$C_6$-alkyl)$_2$ or -O-$SiR_8R_9R_{10}$;
wherein
"$C_1$-$C_6$-alkyl" means a linear or branched, cyclic or open-chain saturated hydrocarbon residue with 1 to 6 carbon atoms, possibly substituted with 1 to 6 halogen atoms, identical or different, selected from F, Cl and Br, and
"$C_1$-$C_6$-alkylene" means a linear or branched, cyclic or open-chain saturated hydrocarbon residue with 1 to 6 carbon atoms, possibly substituted with 1 to 6 halogen atoms, identical or different, selected from F, Cl and B.

**11.** Method according to claim 10, **characterised in that** it comprises the step

(e) converting the at least one of the residues $R_1$, $R_2$ and $R_3$ which is -O-$C_1$-$C_6$-alkyl, -O-$C_1$-$C_6$-alkylenephenyl, -OCO-$C_1$-$C_6$-alkyl, -$OCO_2$-$C_1$-$C_6$-alkyl, -OCO-N($C_1$-$C_6$-alkyl)$_2$ or -O-$SiR_8R_9R_{10}$ into an -OH group.

## Revendications

**1.** Procédé pour l'isolement d'un stéréo-isomère d'un mélange comprenant les deux stéréo-isomères des formules générales (I-A) et (I-A') et/ou les deux stéréo-isomères des formules générales (I-B) et (I-B')

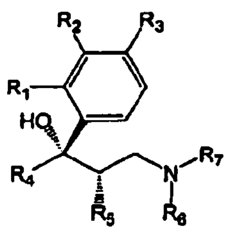
(I-A)

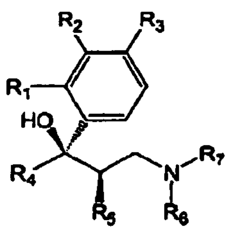
(I-A')

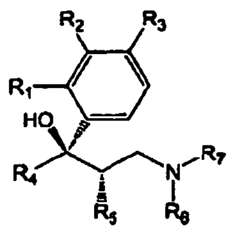
(I-B)

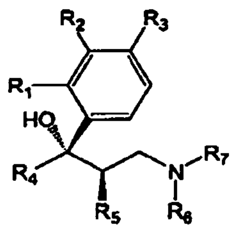
(I-B')

dans lesquelles

$R_1$, $R_2$ et $R_3$ sont identiques ou différents et choisis dans le groupe constitué par -H, -F, -Cl, -$C_1$-$C_6$-alkyle, -S-$C_1$-$C_6$-alkyle, -OH, -O-$C_1$-$C_6$-alkyle, -$OC_1$-$C_6$-alkylènephényle, -OCO-$C_1$-$C_6$-alkyle, -$OCO_2$-$C_1$-$C_6$-alkyle, -OCON($C_1$-$C_6$-alkyle)$_2$ et -O-$SiR_8R_9R_{10}$ (où $R_8$, $R_9$ et $R_{10}$ sont identiques ou différents et représentent -$C_1$-$C_6$-alkyle ou -phényle) ;
$R_4$ représente -H ou -$C_1$-$C_6$-alkyle ;
$R_5$ représente -$C_1$-$C_6$-alkyle ; et
$R_6$ et $R_7$ sont identiques ou différents et représentent -H ou -$C_1$-$C_6$-alkyle ; où"$C_1$-$C_6$-alkyle" signifie un radical hydrocarboné linéaire ou ramifié, cyclique ou à chaîne ouverte, saturé, comprenant 1 à 6 atomes de carbone, le cas échéant substitué par 1 à 6 atomes d'halogène, identiques ou différents et choisis parmi -F, -Cl et -Br, et "$C_1$-$C_6$-alkylène" signifie un radical hydrocarboné linéaire ou ramifié, cyclique ou à chaîne ouverte, saturé, comprenant 1 à 6 atomes de carbone, le cas échéant substitué par 1 à 6 atomes d'halogène, identiques ou différents et choisis parmi -F, -Cl et -Br ; ou leurs sels avec des acides organiques ou inorganiques ; comprenant les étapes :

(a) influence du rapport de mélange des stéréo-isomères dans le mélange, de manière telle qu'au moins un des stéréo-isomères se trouve en un excès d'énantiomère, et
(b) addition d'acide phosphorique dans des conditions qui provoquent la précipitation du stéréo-isomère se trouvant en excès d'énantiomère d'une solution sous forme de phosphate, d'hydrogénophosphate ou de dihydrogénophosphate.

**2.** Procédé selon la revendication 1, **caractérisé en ce que** l'influence dans l'étape (a) a lieu

- par addition du stéréo-isomère, qui doit se trouver en excès, ou
- par une synthèse énantiosélective des stéréo-isomères.

**3.** Procédé selon la revendication 1 ou 2, **caractérisé en ce que** l'excès d'énantiomère est d'au moins 1,0% d'ee.

**4.** Procédé selon l'une quelconque des revendications précédentes, **caractérisé en ce que** $R_1$ et $R_3$ représentent à chaque fois -H et $R_2$ représente $-OCH_3$.

**5.** Procédé selon l'une quelconque des revendications précédentes, **caractérisé en ce que** $R_4$ représente $-CH_2CH_3$ et $R_5$, $R_6$ et $R_7$ représentent à chaque fois $-CH_3$.

**6.** Procédé selon l'une quelconque des revendications précédentes, **caractérisé en ce qu'**il comprend l'étape :

(c) séparation du sel précipité obtenu dans l'étape (b) de la solution surnageante.

**7.** Procédé selon la revendication 6, **caractérisé en ce qu'**il comprend l'étape :

(d) transformation du sel séparé dans l'étape (c) en chlorhydrate.

**8.** Sel d'addition d'un stéréo-isomère de formule générale (I-A), (I-A'), (I-B) ou (I-B') défini comme dans la revendication 1, 4 ou 5 et d'acide phosphorique.

**9.** Sel d'addition selon la revendication 8, choisi dans le groupe constitué par :

- phosphate de (2R,3R)-1-diméthylamino-3-(3-méthoxyphényl)-2-méthylpentan-3-ol,
- hydrogénophosphate de (2R,3R)-1-diméthylamino-3-(3-méthoxyphényl)-2-méthylpentan-3-ol,
- dihydrogénophosphate de (2R,3R)-1-diméthylamino-3-(3-méthoxyphényl)-2-méthylpentan-3-ol,
- phosphate de (2S,3S)-1-diméthylamino-3-(3-méthoxyphényl)-2-méthylpentan-3-ol,
- hydrogénophosphate de (2S,3S)-1-diméthylamino-3-(3-méthoxyphényl)-2-méthylpentan-3-ol,
- dihydrogénophosphate de (2S,3S)-1-diméthylamino-3-(3-méthoxyphényl)-2-méthylpentan-3-ol,
- phosphate de (2R,3S)-1-diméthylamino-3-(3-méthoxyphényl)-2-méthylpentan-3-ol,
- hydrogénophosphate de (2R,3S)-1-diméthylamino-3-(3-méthoxyphényl)-2-méthylpentan-3-ol,
- dihydrogénophosphate de (2R,3S)-1-diméthylamino-3-(3-méthoxyphényl)-2-méthylpentan-3-ol,
- phosphate de (2S,3R)-1-diméthylamino-3-(3-méthoxyphényl)-2-méthylpentan-3-ol,
- hydrogénophosphate de (2S,3R)-1-diméthylamino-3-(3-méthoxyphényl)-2-méthylpentan-3-ol et
- dihydrogénophosphate de (2S,3R)-1-diméthylamino-3-(3-méthoxyphényl)-2-méthylpentan-3-ol.

**10.** Procédé pour la préparation d'un stéréo-isomère de formule générale (I-A), (I-A'), (I-B) ou (I-B')

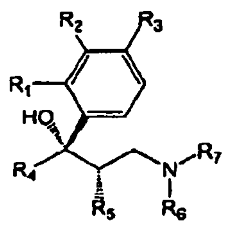
(I-A)

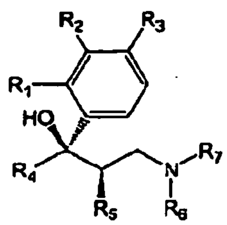
(I-A')

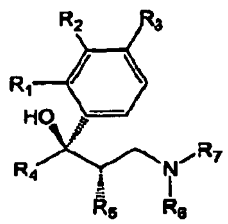
(I-B)

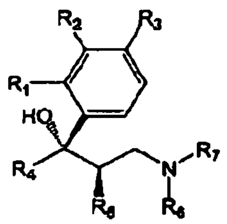
(I-B')

dans lesquelles
$R_1$, $R_2$ et $R_3$ sont identiques ou différents et choisis dans le groupe constitué par -H, -F, -Cl, $-C_1$-$C_6$-alkyle, -S-$C_1$-$C_6$-alkyle et -OH, à condition qu'au moins un des radicaux
$R_1$, $R_2$ et $R_3$ représente -OH ;
$R_4$ représente -H ou $-C_1$-$C_6$-alkyle ;
$R_5$ représente $-C_1$-$C_6$-alkyle ; et
$R_6$ et $R_7$ sont identiques ou différents et
représentent -H ou $-C_1$-$C_6$-alkyle ;
ou leurs sels avec des acides organiques ou inorganiques ;
comprenant le procédé selon l'une quelconque des revendications 1 à 9, au moins un des radicaux $R_1$, $R_2$ et $R_3$ représentant $-O-C_1$-$C_6$-alkyle, $-O-C_1$-$C_6$-alkylènephényle, $-OCO-C_1$-$C_6$-alkyle, $-OCO_2-C_1$-$C_6$-alkyle, -OCON($C_1$-$C_6$-alkyle) ou $-O-SiR_8R_9R_{10}$ ;
où

"$C_1$-$C_6$-alkyle" signifie un radical hydrocarboné linéaire ou ramifié, cyclique ou à chaîne ouverte, saturé, comprenant 1 à 6 atomes de carbone, le cas échéant substitué par 1 à 6 atomes d'halogène, identiques ou différents et choisis parmi -F, -Cl et -Br, et
"$C_1$-$C_6$-alkylène" signifie un radical hydrocarboné linéaire ou ramifié, cyclique ou à chaîne ouverte, saturé, comprenant 1 à 6 atomes de carbone, le cas échéant substitué par 1 à 6 atomes d'halogène, identiques ou différents et choisis parmi -F, -Cl et -Br.

**11.** Procédé selon la revendication 10, **caractérisé en ce qu'**il comprend l'étape :

(e) transformation dudit au moins un des radicaux $R_1$, $R_2$ et $R_3$, qui représente -O-$C_1$-$C_6$-alkyle, -O-$C_1$-$C_6$-alkylènephényle, -OCO-$C_1$-$C_6$-alkyle, -$OCO_2$-$C_1$-$C_6$-alkyle, -OCO-N($C_1$-$C_6$-alkyle)$_2$ ou -O-Si$R_8R_9R_{10}$, en un groupe -OH.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 693475 A
- EP 0693475 A1


### In der Beschreibung aufgeführte Nicht-Patentliteratur


- **J. R. DEHLI et al.** *Tetrahedron: Asymmetry,* 2001, vol. 12 (10), 1485-1492
- **L. ANGLIOLINI et al.** *Tetrahedron,* 1969, vol. 25, 4211-4216
- **I. OJIMA.** Catalytic Asymmetric Synthesis. Wiley VCH
- **R.A. AITKEN et al.** Asymmetric Synthesis. CRC Press
- **L.A. PAQUETTE.** Handbook of Reagents for Organic Synthesis : Chiral Reagents for Asymmetric Synthesis. John Wiley & Sons
- **H.B. KAGAN.** Asymmetric Synthesis. Thieme Medical Pub,
- **W. CARRUTHERS.** Modem Methods of Organic Synthesis. Cambridge University Press
- **R.S. ATKINSON.** Stereoselective Synthesis. John Wiley & Sons